# EUROPEAN PATENT SPECIFICATION

(11) **EP 3 941 360 B1**
(45) Date of publication and mention of the grant of the patent: **24.06.2026**
(21) Application number: 20718103.3
(22) Date of filing: 20.03.2020
(51) Int. Cl.: A61B 17/00, A61B 17/34

(54) **SEALANT DELIVERY APPARATUS, AND SYSTEM AND METHOD FOR PREPARING SAME, FOR USE IN A LUNG PROCEDURE**
VORRICHTUNG ZUR ABGABE EINES VERSIEGELUNGSMITTELS SOWIE EIN SYSTEM UND VERFAHREN ZUR HERSTELLUNG DAVON ZUR VERWENDUNG IN EINEM LUNGENVERFAHREN
APPAREIL DE DISTRIBUTION DE PRODUIT D'ÉTANCHÉITÉ, ET SYSTÈME ET PROCÉDÉ DE PRÉPARATION DE CELUI-CI, DESTINÉS À ÊTRE UTILISÉS DANS UNE PROCÉDURE PULMONAIRE

(30) Priority: 22.03.2019 US 201962822490 P
(43) Date of publication of application: 26.01.2022
(73) Proprietor: C. R. Bard, Inc., Franklin Lakes, NJ 07417 (US)
(72) Inventor: ADDISON, Jordan P., Franklin Lakes, NJ 07417 (US); GLASPIE, Koltin K., Franklin Lakes, NJ 07417 (US); STORM, Heather A., Franklin Lakes, NJ 07417 (US)
(74) Representative: Hoffmann Eitle
(86) International application number: PCT/US2020/023772
(87) International publication number: WO 2020/197969

(56) References cited:
- EP-A1- 0 292 472
- WO-A1-2014/077877
- WO-A1-2018/150375
- WO-A1-2019/050169
- WO-A1-99/32173
- KR-B1- 101 909 259
- US-A1- 2005 027 240
- US-A1- 2005 096 588
- US-A1- 2006 161 110
- US-A1- 2009 198 177
- US-A1- 2011 282 383
- US-A1- 2017 100 115
- US-A1- 2017 273 675
- US-B2- 8 460 235

## Description

### Cross-Reference To Related Applications

This application claims priority to U.S. provisional patent application serial no. 62/822,490 entitled "LUNG BIOPSY FLOWABLE SEALANT DELIVERY SYSTEM" filed March 22, 2019.

### Technical Field

The present disclosure relates to a lung access procedure, such as a lung biopsy, and, more particularly, to a sealant delivery apparatus, and system and method for preparing the same, for use associated with a lung procedure to aid in preventing pneumothorax.

### Background Art

Pneumothorax is a problematic complication of the lung biopsy procedure where air or fluid is allowed to pass into the pleural space as a result of the puncture of the parietal pleura and visceral pleura. Pneumothorax and, more so, pneumothorax requiring chest tube placement, are significant concerns for clinicians performing, and patients undergoing, percutaneous lung biopsies. The incidence of pneumothorax in patients undergoing percutaneous lung biopsy has been reported to be anywhere from 9-54%, with an average of around 15%. On average, 6.6% of all percutaneous lung biopsies result in pneumothorax requiring a chest tube to be placed, which results in an average hospital stay of 2.7 days.

Factors that increase the risk of pneumothorax include increased patient age, obstructive lung disease, increased depth of a lesion, multiple pleural passes, increased time that an access needle lies across the pleura, and traversal of a fissure. Pneumothorax may occur during or immediately after the procedure, which is why typically a CT scan of the region is performed following removal of the needle. Other, less common, complications of percutaneous lung biopsy include hemoptysis (coughing up blood), hemothorax (a type of pleural effusion in which blood accumulates in the pleural cavity), infection, and air embolism.

It has been suggested that approximately 30% of lung biopsies result in some form of pneumothorax that makes deploying a plug after the biopsy difficult or impossible. Current sealants on the market are deployed through a coaxial cannula after a biopsy is performed which may be before or after a pneumothorax forms.

What is needed in the art is a sealant delivery apparatus with the ability to deposit sealant components into the pleural space prior to a lung procedure, such as a biopsy, to aid in preventing pneumothorax, and a system and method for preparing the same.

WO 2014/077877 A1 discloses a bioadhesive mixing assembly including first and second syringes and an adapter. The first syringe includes first and second chambers holding a first sealant component and an activator, respectively. The second syringe includes third and fourth chambers holding a second sealant component and one of an activator or a third sealant component, respectively. The adapter is mounted to the first syringe and includes first and second channels in flow communication with the first and second chambers, a first seal member providing sealed access to the first and second channels, first and second needles connected in flow communication with the third and fourth chambers, and a second seal member enclosing the first and second needles. Connecting the adapter to the second syringe punctures the first and second seal members with the first and second syringes to create flow communication between the first and third chambers and the second and fourth chambers.

US 8460235 B2 discloses a dispensing assembly for two components with double syringe and mixer.

### Summary of Invention

The present disclosure provides a sealant delivery apparatus with the ability to deposit sealant components into the pleural space prior to a lung procedure, such as a biopsy, to aid in preventing pneumothorax, and a system and method for preparing the same. Claim 1 is directed to a system for preparing a sealant delivery apparatus for use in a lung access procedure. Claim 7 is directed to a method for preparing a sealant delivery apparatus for use in a lung access procedure. The dependent claims refer to preferred embodiments.

The disclosure, in one form not having all features of the independent claims, is directed to a sealant delivery apparatus for use in a lung access procedure to aid in preventing pneumothorax. The sealant delivery apparatus includes a sealant applicator device and an injection needle assembly. The sealant applicator device is configured to separately carry each of a first sealant component of a multi-component sealant and a second sealant component of the multi-component sealant. The sealant applicator device has at least one output port. The injection needle assembly has a hub and an elongate hollow stylet that extends distally from the hub. The hub is configured for removable connection to the sealant applicator device. The elongate hollow stylet is configured to facilitate fluid communication with the at least one output port of the sealant applicator device to receive the multi-component sealant. The elongate hollow stylet has a proximal portion and a distal portion. The distal portion has a closed distal end and a plurality of side ports proximal to the closed distal end. The elongate hollow stylet has a side wall that surrounds a lumen, wherein the plurality of side ports radially extend from the lumen and through the side wall of the elongate hollow stylet in the distal portion. The plurality of side ports are in fluid communication with the at least one output port of the sealant applicator device.

The invention is directed to a system for preparing a sealant delivery apparatus for use in a lung access procedure of claim 1. The system in particular includes a first pair of syringes associated with the sealant delivery apparatus, a second pair of syringes, and a coupling mechanism. The first pair of syringes includes a first actuator, a first component chamber having a first component port, and a second component chamber having a second component port. The first actuator includes a first piston and a second piston. The first piston is positioned in the first component chamber and the second piston is positioned in the second component chamber. The first component chamber initially contains a first powder component of a first sealant component of a multi-component sealant, and the second component chamber initially contains a second powder component of the second sealant component of the multi-component sealant. The second pair of syringes includes a second actuator, a first component reservoir having a first transfer port, and a second component reservoir having a second transfer port. The second actuator includes a third piston and a fourth piston. The third piston is positioned in the first component reservoir and the fourth piston is positioned in the second component reservoir. The first component reservoir initially contains a first fluid component of the first sealant component of the multi-component sealant, and the second component reservoir initially contains a second fluid component of the second sealant component of the multi-component sealant. The coupling mechanism has a first coupling end and a second coupling end. The first coupling end is configured to releasably connect to the first pair of syringes and the second coupling end is configured to releasably connect to the second pair of syringes. The coupling mechanism has a first passage and a second passage. The first passage is configured to facilitate fluid communication between the first transfer port of the second pair of syringes and the first component port of the first pair of syringes, and the second passage is configured to facilitate fluid communication between the second transfer port of the second pair of syringes and the second component port of the first pair of syringes.

The invention is directed to a method for preparing a sealant delivery apparatus for use in a lung access procedure of claim 8. The method includes in particular providing a first pair of syringes that includes a first actuator, a first component chamber having a first component port, and a second component chamber having a second component port, and wherein the first actuator includes a first piston and a second piston, the first piston being positioned in the first component chamber and the second piston being positioned in the second component chamber, the first component chamber containing a first powder or solution component of a first sealant component of a multi-component sealant, and the second component chamber containing a second powder or solution component of the second sealant component of the multi-component sealant; providing a second pair of syringes that includes a second actuator, a first component reservoir having a first transfer port, and a second component reservoir having a second transfer port, and wherein the second actuator includes a third piston and a fourth piston, the third piston being positioned in the first component reservoir and the fourth piston being positioned in the second component reservoir, the first component reservoir containing a first fluid component of the first sealant component of the multi-component sealant, and the second component reservoir containing a second fluid component of the second sealant component of the multi-component sealant; and providing a coupling mechanism having a first coupling end and a second coupling end, the first coupling end being releasably connectable to the first pair of syringes and the second coupling end being releasably connectable to the second pair of syringes, the coupling mechanism having a first passage and a second passage, wherein the first passage facilitates fluid communication between the first transfer port of the second pair of syringes and the first component port of the first pair of syringes, and wherein the second passage facilitates fluid communication between the second transfer port of the second pair of syringes and the second component port of the first pair of syringes.

An advantage of the present invention is that the invention improves upon typical solutions in that the multi-component sealant seals the pneumothorax region before the biopsy, rather than after the biopsy.

Another advantage of the present invention is that the invention improves over typical pneumothorax prevention devices in that, since the injection needle assembly is deployed at the beginning of the procedure rather than at the very end of a procedure, the flowable multi-component sealant, e.g., polymer, is able to be delivered and integrate into the spaces between tissues, whereas polymer plugs, for example, only occupy the space that they were cast and thus may result in a less effective seal.

Another advantage of the present invention is that the invention also requires no measuring of where the needle is in relation to the pleura beyond what physicians currently do. The solution of the present invention should be able to be seamlessly integrated into the lung access procedure by preparation of the flowable multi-component sealant, and dispensing the flowable multi-component sealant from the elongate hollow stylet of the injection needle assembly as the elongate hollow stylet is advanced across the pleura.

### Brief Description of Drawings

The above-mentioned and other features and advantages of this disclosure, and the manner of attaining them, will become more apparent and the invention will be better understood by reference to the following description of embodiments of the disclosuretaken in conjunction with the accompanying drawings, wherein:
Fig. 1 is a front view of the sealant delivery apparatus in accordance with an aspect of the present disclosure not having all features of claim 1;
Fig. 2 is an exploded view of the sealant delivery apparatus of Fig. 1;
Fig. 3 is a side view of the sealant delivery device of the sealant delivery apparatus of Fig. 1, with the actuator retracted;
Fig. 4 is a section view of the sealant delivery device depicted in Fig. 3, taken along line 4-4 of Fig. 3, with the actuator retracted, and showing a first piston in a first component chamber proximal to a first sealant component of a multi-component sealant, and a second piston in a second component chamber proximal to a second sealant component of the multi-component sealant;
Fig. 5 is a perspective view of a distal portion of an elongate stylet of an injection needle assembly of the sealant delivery apparatus Figs. 1 and 2;
Fig. 6 is a side view of the distal portion of the elongate stylet of the injection needle assembly of the sealant delivery apparatus Figs. 1, 2, and 5;
Fig. 7 is a section view of the distal portion of the elongate stylet of the injection needle assembly of the sealant delivery apparatus Figs. 1, 2, 5, and 6, taken along line 7-7 of Fig. 6;
Fig. 8 is a front view of a system for preparing the sealant delivery apparatus of Figs. 1 and 2 for use in a lung access procedure, including the first pair of syringes and the coupling mechanism of the sealant delivery apparatus, and further including a second pair of syringes coupled to the first pair of syringes by the coupling mechanism;
Fig. 9 is an exploded view of the system of Fig. 8;
Fig. 10 is a side view of the system of Fig. 8, with the first actuator of the first pair of syringes retracted, and with the second actuator of a second pair of syringes retracted;

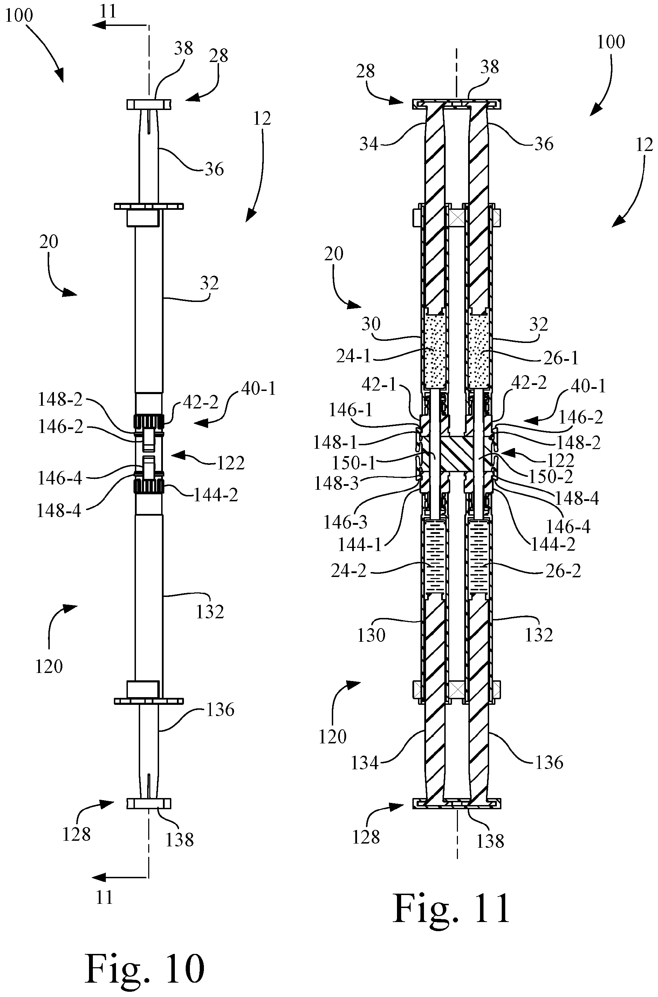

Fig. 11 is a section view of the system depicted in Fig. 10, taken along line 11-11 of Fig. 10, with the first actuator and the second actuator retracted, wherein a first component chamber of the first pair of syringes initially contains a powder or solution component of the first sealant component of the multi-component sealant, a second component chamber of the first pair of syringes initially contains a powder or solution component of the second sealant component of the multi-component sealant, a first component reservoir of the second pair of syringes initially contains a fluid component of the second sealant component of the multi-component sealant, and a second component reservoir of the second pair of syringes initially contains a fluid component of the second sealant component of the multi-component sealant; and
Fig. 12 is a flowchart of a method for preparing the sealant delivery apparatus of Figs. 1 and 2, and more particularly the pair of syringes of the sealant delivery apparatus, for use in a lung access procedure.

Corresponding reference characters indicate corresponding parts throughout the several views. The exemplifications set out herein illustrate at least one embodiment of the invention, and such exemplifications are not to be construed as limiting the scope of the invention in any manner.

### Description of Embodiments

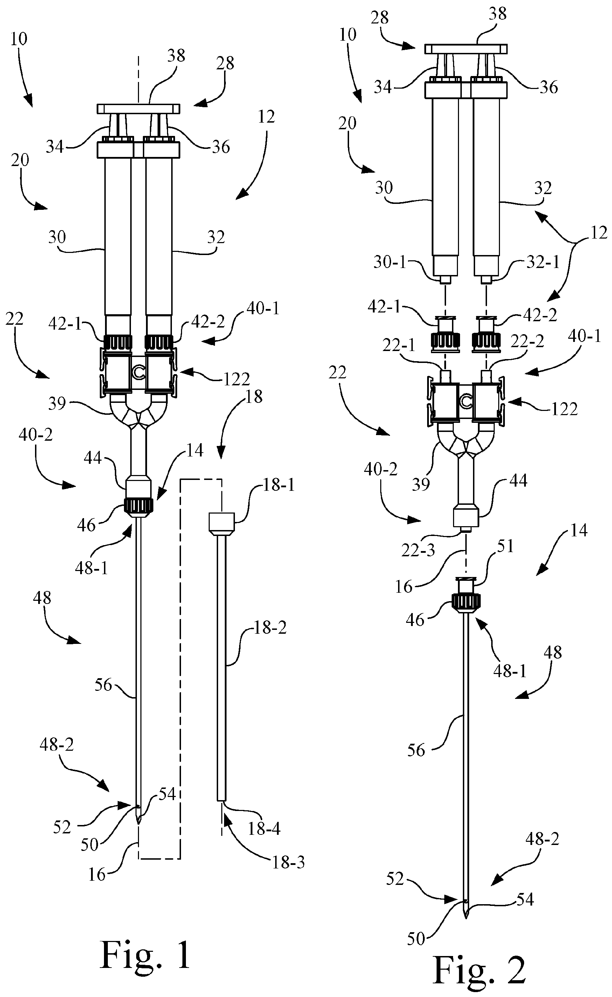

Referring now to the drawings, and more particularly to Figs. 1 and 2, there is shown a sealant delivery apparatus 10 in accordance with an aspect of the present disclosure not having all features of claim 1, for use in a lung access procedure to aid in preventing pneumothorax.

Sealant delivery apparatus 10 includes a sealant applicator device 12 and an injection needle assembly 14. Injection needle assembly 14 defines a longitudinal axis 16. When assembled, sealant delivery apparatus 10 has a configuration to facilitate delivery of a flowable multi-component sealant to an injection site, wherein the multi-component sealant may be injected while the needle portion of injection needle assembly 14 crosses the two layers of the pleura, i.e., the parietal and visceral pleura. Injection needle assembly 14 is configured to puncture a patient's tissue and create an access path, e.g., a proposed biopsy tract, in the tissue at least through the pleura of the patient, and thus allows for the two layers of the pleura to be sealed, e.g., prior to a lung biopsy, by the curing of the multi-component sealant at the delivery site so that air cannot leak between the two layers and cause a pneumothorax. It is noted that the multi-component sealant also may be deposited in other regions of the access path, such as in the subcutaneous tissue and/or lung parenchyma.

Optionally, sealant delivery apparatus 10 may be used in conjunction with an introducer cannula 18, wherein introducer cannula 18 may facilitate withdrawal of injection needle assembly 14 of sealant delivery apparatus 10 from the patient, while introducer cannula 18 may remain in place to maintain the access path in the patient's tissue to the site, such as for example, to receive and guide a second medical instrument, such as a biopsy device, to the site where the biopsy is to be performed.

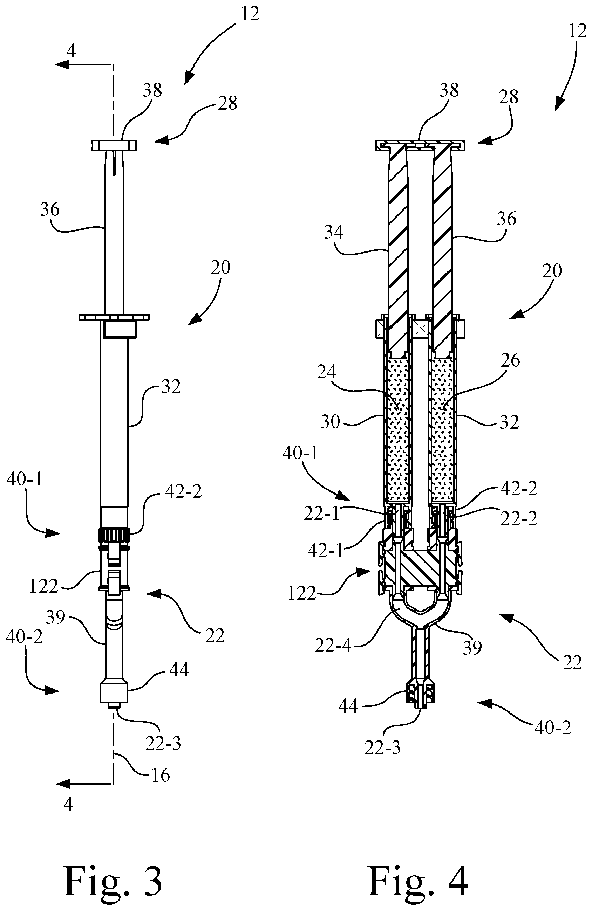

Referring also to Figs. 3 and 4, sealant applicator device 12 includes a pair of syringes 20 and a manifold 22. Sealant applicator device 12 is configured to separately carry each of a first sealant component 24 of the multi-component sealant and a second sealant component 26 of the multi-component sealant. The first sealant component 24 may include, for example, at least two N-hydroxysuccinimide (NHS) ester groups, and the second sealant component 26 may include, for example, at least two amine groups. For example, the first sealant component 24 may be a solution containing polyethylene glycol (PEG) succinimidyl succinate and the second sealant component 26 may be a solution containing albumin and/or polyethylenimine (PEI). In the present embodiment, the first sealant component 24 and the second sealant component 26 are combined and mixed at manifold 22.

The pair of syringes 20 includes an actuator 28, a first component chamber 30, and a second component chamber 32. First component chamber 30 may be, for example, a cylindrical tube that is configured to carry the first sealant component 24 of the multi-component sealant. First component chamber 30 has a first component port 30-1. Second component chamber 32 also may be, for example, a cylindrical tube that is configured to carry the second sealant component 26 of the multi-component sealant. Second component chamber 32 has a second component port 32-1. First component chamber 30 and second component chamber 32 are arranged in a longitudinally parallel arrangement.

Actuator 28 includes a first piston 34, a second piston 36, and a handle 38. Handle 38 is in the form of a link member that perpendicularly extends between, and is connected to, each of first piston 34 and second piston 36 to facilitate simultaneous movement of first piston 34 and second piston 36 with the depression or retraction of handle 38. First piston 34 is in the form of a plunger that is positioned in first component chamber 30 proximal to the first sealant component 24, and second piston 36 is in the form of a plunger that is positioned in second component chamber 32 proximal to the second sealant component 26.

Referring to Figs. 1-4, in the present embodiment not having all features of the independent claims, manifold 22 is removably connectable at a first coupling end 40-1 to the pair of syringes 20 of sealant applicator device 12 by a pair of connecters 42-1, 42-2, and is removably connectable at a second end 40-2 to injection needle assembly 14 by a connector 44. More particularly, as best shown in Fig. 4, manifold 22 includes a Y-connector 39 releasably connected to a coupling mechanism 122, wherein coupling mechanism 122 serves as a detachable extension of the two-port portion of Y-connector 39. Thus, Y-connector 39 (and more particularly, coupling mechanism 122) is removably connectable at first coupling end 40-1 to the pair of syringes 20 of sealant applicator device 12 by the pair of connecters 42-1, 42-2, and Y-connector 39 is removably connectable at a second end 40-2 to injection needle assembly 14 by a connector 44.

The pair of connecters 42-1, 42-2 may form a threaded, or alternatively a snap-fit, connection with the pair of syringes 20 of sealant applicator device 12. Likewise, connector 44 may form a threaded, or alternatively a snap-fit, connection with injection needle assembly 14.

Those skilled in the art will recognize that the pair of connecters 42-1, 42-2 may be included as a part of the pair of syringes 20, and releasably connectable to manifold 22. Alternatively, the pair of connecters 42-1, 42-2 may be included as a part of manifold 22, and releasably connectable to the pair of syringes 20. Further, alternatively, the pair of connecters 42-1, 42-2 may be included as separate parts, each of which is separately connectable to each of the pair of syringes 20 and the manifold 22.

Manifold 22 has a first input port 22-1, a second input port 22-2, and at least one output port, which in the present embodiment is a single output port, referenced hereinafter as output port 22-3. As best shown in Fig. 4, in the present embodiment, coupling mechanism 122 of manifold 22 has individual passages in fluid communication with an internal Y-passageway 22-4 of Y-connector 39. In the present embodiment, coupling mechanism 122 of manifold 22 directly includes first input port 22-1 and second input port 22-2, and Y-connector 39 of manifold 22 directly includes output port 22-3. Thus, by virtue of Y-connector 39, the at least one output port, e.g., output port 22-3, is in fluid communication with each of first input port 22-1 and second input port 22-2. Also, when manifold 22 is connected to the pair of syringes 20 of sealant applicator device 12, then first input port 22-1 is in fluid communication with first component chamber 30 and second input port 22-2 is in fluid communication with second component chamber 32.

Referring to Figs. 1 and 2, injection needle assembly 14 includes a hub 46 and an elongate hollow stylet 48 that extends distally from hub 46. Elongate hollow stylet 48 has a proximal portion 48-1 and a distal portion 48-2. Hub 46 is fixedly attached, e.g., through overmolding, adhesive and/or pressed fit, to proximal portion 48-1 of elongate hollow stylet 48. Hub 46 is configured for removable connection to sealant applicator device 12. More particularly, hub 46 of injection needle assembly 14 may include a connector 51 (see Fig. 2) that may form a threaded, or alternatively a snap-fit, connection with connector 44 at second end 40-2 of manifold 40 of sealant applicator device 12.

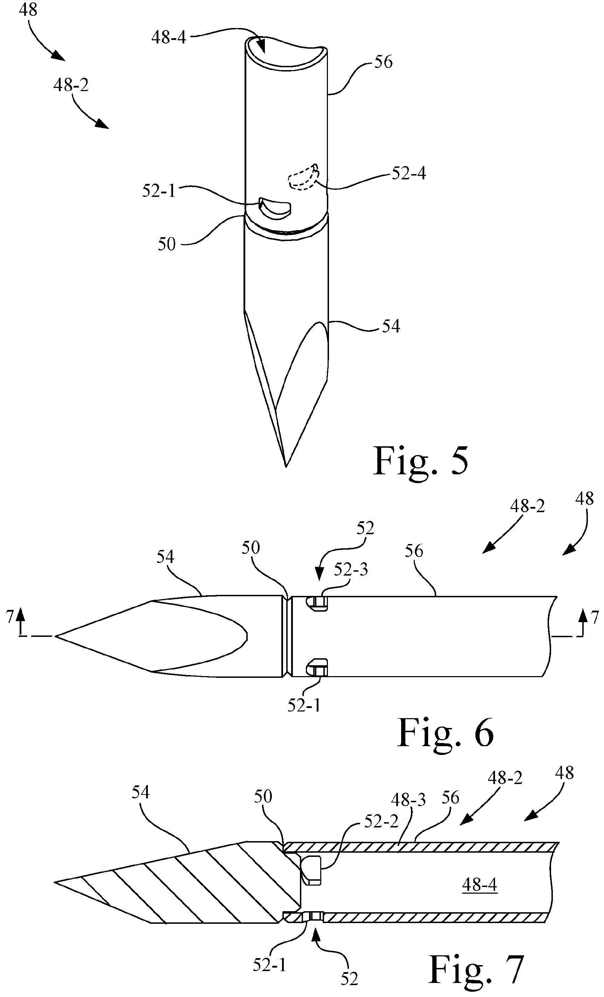

Elongate hollow stylet 48 of injection needle assembly 14 is configured to facilitate fluid communication with the at least one output port, e.g., output port 22-3 of manifold 22 of sealant applicator device 12 so as to receive the multi-component sealant from sealant applicator device 12. Referring also to Figs. 5-7, distal portion 48-2 has a closed distal end 50 and a plurality of side ports 52 (e.g., at least three) proximal to closed distal end 50. Closed distal end 50 of elongate hollow stylet 48 may be, for example, a closed stylet needle tip 54.

Elongate hollow stylet 48, may be constructed, for example, by an elongate cannula 56 being fixedly connected to the closed stylet needle tip 54, with the plurality of side ports 52 being located in elongate cannula 56 immediately proximal to closed stylet needle tip 54. More particularly, elongate cannula 56 of elongate hollow stylet 48 defines a side wall 48-3 that surrounds a lumen 48-4 of elongate hollow stylet 48, wherein the plurality of side ports 52 radially extend from lumen 48-4 and through side wall 48-3 of elongate hollow stylet 48 in distal portion 48-2. Closed stylet needle tip 54 is defined, at least in part, by closed distal end 50 of elongate hollow stylet 48, wherein closed stylet needle tip 54 is attached, e.g., welded, press fit, or with adhesive, to elongate cannula 56 to distally close lumen 48-4 of elongate cannula 56 of elongate hollow stylet 48. Stated differently, closed stylet needle tip 54 terminates a distal extent of lumen 48-4 of elongate hollow stylet 48. When sealant delivery apparatus 10 is assembled, the plurality of side ports 52 are in fluid communication with the at least one output port, e.g., output port 22-3, of manifold 22 of sealant applicator device 12 by way of lumen 48-4 of elongate hollow stylet 48.

In the present embodiment, the plurality of side ports 52 in distal portion 48-2 of elongate hollow stylet 48 includes a side port 52-1, a side port 52-2, and a side port 52-3, which are located in distal portion 48-2 and arranged, e.g., in a ring pattern, around a perimeter of elongate hollow stylet 48, such as in 120 degree increments. In one application, for example, it is preferred that the plurality of side ports 52 include at least three side ports (e.g., 3 to 7 side ports) to have the at least three side ports near, but proximal to, closed stylet needle tip 54 such that the flowable multi-component sealant may be delivered 360 degrees around elongate hollow stylet 48, so as to seal around the perforation/close to the perforation created by closed stylet needle tip 54. As another example, it is contemplated that in some applications it may be desirable to have the plurality of side ports 52 configured as two, or two pairs, of diametrically opposed side ports.

Optionally, as shown by phantom lines in Fig. 5, it is further contemplated that the plurality of side ports 52 may include at least two longitudinally spaced side ports, such as for example, a side port 52-4 longitudinally spaced (e.g. 1 to 3 millimeters) proximal to side port 52-1. For example, the plurality of side ports 52 may include two rings of three side ports arranged around a perimeter of elongate hollow stylet 48, wherein the two rings of three side ports are longitudinally spaced in distal portion 48-2 of elongate hollow stylet 48.

Referring again to Fig. 1, injection needle assembly 14 may be used in conjunction with the introducer cannula 18 (also sometimes referred to in the art as a coaxial introducer needle) to allow elongate hollow stylet 48 to be removed from introducer cannula 18, while maintaining access to the procedure site with the coaxial introducer needle. In other words, sealant delivery apparatus 10 may be removed from introducer cannula 18 and replaced with a variety of other instruments, such as a biopsy device or another stylet. In the present embodiment, introducer cannula 18 has a coaxial hub 18-1, a coaxial cannula 18-2, a cannula lumen 18-3 and a distal annular rim 18-4. Cannula lumen 18-3, e.g., is configured, e.g., having a cylindrical shape, to receive elongate hollow stylet 48 of injection needle assembly 14. When elongate hollow stylet 48 of injection needle assembly 14 is fully inserted into cannula lumen 18-3 of introducer cannula 18 (e.g., distal movement of elongate hollow stylet 48 is stopped by contact of hub 46 of injection needle assembly 14 with coaxial hub 18-1 of introducer cannula 18, the plurality of side ports 52 of elongate hollow stylet 48 are located distal to the distal annular rim 18-4 of introducer cannula 18.

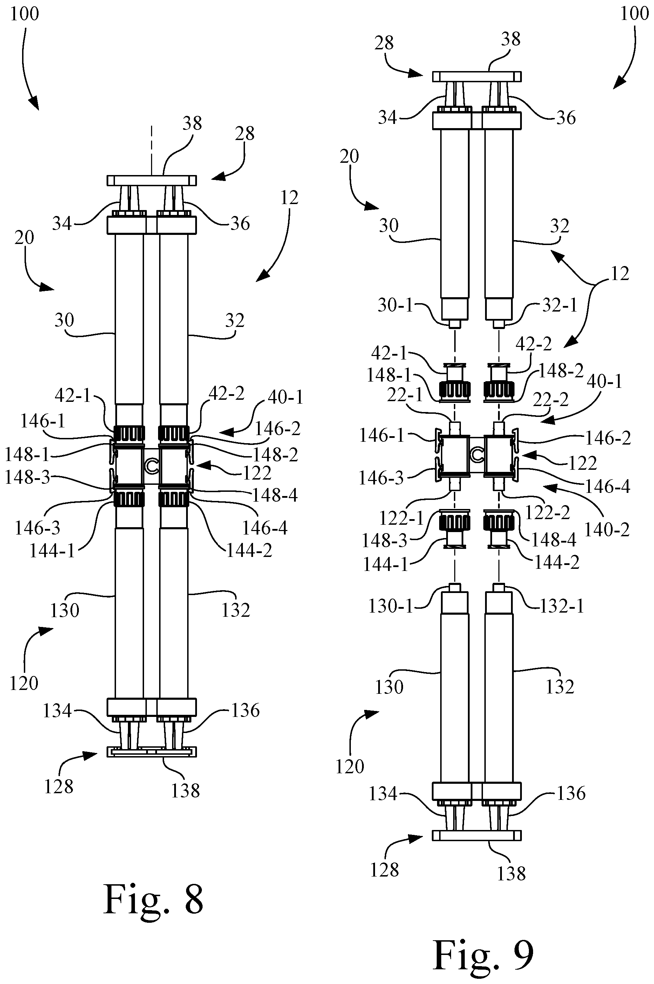

Referring to Figs. 8-11, there is show a system 100 for preparing sealant delivery apparatus 10 for use in a lung access procedure. More particularly, system 100 is used to mix various chemical agents, which when combined will result in the first sealant component 24 and the second sealant component 26 of the multi-component sealant depicted in Fig. 4. System 100 generally includes the first pair of syringes 20, a second pair of syringes 120, and coupling mechanism 122.

As described above, the first pair of syringes 20 is configured having first component chamber 30, second component chamber 32, and the first actuator 28 having first piston 34 and second piston 36. However, initially (e.g., as a deliverable from the manufacturer), first component chamber 30 does not yet contain the prepared first sealant component 24 and second component chamber 32 does not yet contain the prepared second sealant component 26, as depicted in Fig. 4. Rather, referring to Fig. 11, initially, first component chamber 30 initially contains a powder or solution component 24-1 of the first sealant component 24 of the multi-component sealant, and second component chamber 32 initially contains a powder or solution component 26-1 of the second sealant component 26 of the multi-component sealant. As used herein, each solution forming component 24-1 and/or component 26-1 is a solute and solvent combination, and may include, for example, a suspension or hydrogel.

Referring to Figs. 8-11, the second pair of syringes 120 includes a second actuator 128, a first component reservoir 130 having a first transfer port 130-1, and a second component reservoir 132 having a second transfer port 132-1.

Second actuator 128 includes a third piston 134, a fourth piston 136, and a handle 138. Handle 138 is in the form of a link member that perpendicularly extends between, and is connected to, each of third piston 134 and fourth piston 136 to facilitate simultaneous movement of third piston 134 and fourth piston 136 with the depression or retraction of handle 138. Third piston 134 is in the form of a plunger that is positioned in first component reservoir 130 and fourth piston 136 is in the form of a plunger that is positioned in second component reservoir 132. First component reservoir 130 initially contains a fluid component 24-2 of the first sealant component 24 of the multi-component sealant, and second component reservoir 132 initially contains a fluid component 26-2 of the second sealant component 26 of the multi-component sealant. As used herein, fluid component 24-2 and/or fluid component 26-2 may be, or include, water or some other liquid.

Coupling mechanism 122 has a first coupling end 40-1 and a second coupling end 140-2. First coupling end 40-1 is configured to releasably connect to the first pair of syringes 20, and second coupling end 140-2 is configured to releasably connect to the second pair of syringes 120. Referring particularly to Figs. 9 and 11, coupling mechanism 122 includes first input port 22-1 and second input port 22-2 at first coupling end 40-1, and includes a port 122-1 and a port 122-2 at second coupling end 140-2. Coupling mechanism 122 further includes a first passage 150-1 in fluid communication with, and extending between, port 22-1 and port 122-1. Likewise, coupling mechanism 122 includes a second passage 150-2 in fluid communication with, and extending between, port 22-2 and port 122-2.

The pair of connecters 42-1, 42-2 form a threaded, (does not form part of the claimed invention) or alternatively a snap-fit, connection between the first pair of syringes 20 and first coupling end 40-1 of coupling mechanism 122. Likewise, a pair of connecters 144-1, 144-2 form a threaded, (does not form part of the claimed invention) or alternatively a snap-fit, connection between the second pair of syringes 120 and second coupling end 140-2 of coupling mechanism 122.

The pair of connecters 42-1, 42-2 may be included as a part of the first pair of syringes 20, and releasably connectable to coupling mechanism 122 (does not form part of the claimed invention). Alternatively, the pair of connecters 42-1, 42-2 may be included as a part of coupling mechanism 122, and releasably connectable to the first pair of syringes 20 (does not form part of the claimed invention). As a further alternative, the pair of connecters 42-1, 42-2 are included as separate parts, each of which is separately connectable to each of the pair of syringes 20 and coupling mechanism 122.

Likewise, the pair of connecters 144-1, 144-2 may be included as a part of the second pair of syringes 120, and releasably connectable to coupling mechanism 122 (does not form part of the claimed invention). Alternatively, the pair of connecters 144-1, 144-2 may be included as a part of coupling mechanism 122, and releasably connectable to the second pair of syringes 120 (does not form part of the claimed invention). As a further alternative, the pair of connecters 144-1, 144-2 are included as separate parts, each of which is separately connectable to each of the second pair of syringes 120 and coupling mechanism 122.

Referring to Figs. 8-11, first coupling end 40-1 of coupling mechanism 122 includes a first snap latch 146-1 and a second snap latch 146-2. First component chamber 30 of the first pair of syringes 20 (but more particularly connector 42-1) includes a first snap catch 148-1 configured to releasably engage first snap latch 146-1 of coupling mechanism 122. Similarly, second component chamber 32 of the first pair of syringes 20 (but more particularly connector 42-2) includes a second snap catch 148-2 configured to releasably engage second snap latch 146-2 of coupling mechanism 122.

Second coupling end 140-2 of coupling mechanism 122 includes a third snap latch 146-3 and a fourth snap latch 146-4. First component reservoir 130 of the second pair of syringes 120 (but more particularly connector 144-1) includes a third snap catch 148-3 configured to releasably engage third snap latch 146-3 of coupling mechanism 122. Similarly, second component reservoir 132 of the second pair of syringes 120 (but more particularly connector 144-2) has a fourth snap catch 148-4 configured to releasably engage fourth snap latch 146-4 of coupling mechanism 122.

Referring to Figs. 9 and 11, first passage 150-1 of coupling mechanism 122 is configured to facilitate fluid communication between first transfer port 130-1 of the second pair of syringes 120 and first component port 30-1 of the first pair of syringes 20, and second passage 150-2 of coupling mechanism 122 is configured to facilitate fluid communication between second transfer port 132-1 of the second pair of syringes 120 and second component port 32-1 of the first pair of syringes 20.

Referring to Figs. 10 and 11, first actuator 28 of the first pair of syringes 20 is configured to simultaneously move first piston 34 and second piston 36, and second actuator 128 of the second pair of syringes 120 is configured to simultaneously move third piston 134 and fourth piston 136.

Accordingly, by alternatingly operating, e.g., depressing, second actuator 128 and first actuator 28, the second pair of syringes 120 and the first pair of syringes 20 are configured to alternatingly transfer any contents of first component reservoir 130 and second component reservoir 132 to first component chamber 30 and second component chamber 32, respectively, and to transfer any contents of first component chamber 30 and second component chamber 32 to first component reservoir 130 and second component reservoir 132, respectively. For example, the first pair of syringes 20 and the second pair of syringes 120 may be configured to simultaneously: (A) hydrate powder or solution component 24-1 with fluid component 24-2 to form the first sealant component 24 of the multi-component sealant, wherein when fully hydrated, the first sealant component 24 of the multi-component sealant resides in first component chamber 30 of the first pair of syringes 20 (see also Fig. 4), and (B) hydrate powder or solution component 26-1 with fluid component 26-2 to form the second sealant component 26 of the multi-component sealant, wherein when fully hydrated, the second sealant component 26 of the multi-component sealant resides in second component chamber 32 of the first pair of syringes 20, as depicted in Fig. 4.

For example, in a first depression of second actuator 128, third piston 134 and fourth piston 136 simultaneously transfer fluid component 24-2 of the first sealant component 24 through first passage 150-1 into first component chamber 30 carrying powder or solution component 24-1 of the first sealant component 24 and transfers fluid component 26-2 of the second sealant component 26 through second passage 150-2 into second component chamber 32 carrying powder or solution component 26-1 of the second sealant component 26. Thereafter, in a first depression of first actuator 28, first piston 34 and second piston 36 simultaneously transfer the contents of first component chamber 30 and the contents of second component chamber 32 of the first pair of syringes 20 into first component reservoir 130 and second component reservoir 132, respectively, of the second pair of syringes 120. Then, in a second depression of second actuator 128, third piston 134 and fourth piston 136 simultaneously transfer the contents of first component reservoir 130 and second component reservoir 132 of the second pair of syringes 120 into first component chamber 30 and second component chamber 32, respectively, of the first pair of syringes 20. This process may be repeated, as necessary, to ensure the adequate hydration of powder or solution component 24-1 with fluid component 24-2 to form the first sealant component 24 of the multi-component sealant, and to ensure the adequate hydration of powder or solution component 26-1 with fluid component 26-2 to form the second sealant component 26 of the multi-component sealant.

At the conclusion of hydration/mixing, first sealant component 24 resides in first component chamber 30 and second sealant component 26 resides in second component chamber 32 of the first pair of syringes 20. Coupling mechanism 122 may then be decoupled from the first pair of syringes 20, then manifold 22 may be coupled to first pair of syringes 20, and then injection needle assembly 14 may be coupled to the first pair of syringes 20 via manifold 22 to form the sealant delivery apparatus 10, as described above with respect to Figs. 1-7.

Alternatively, at the conclusion of hydration/mixing, coupling mechanism 122 may then be decoupled from the second pair of syringes 120, then manifold 22 may be formed by coupling Y-connector 39 to coupling mechanism 122, and then injection needle assembly 14 may be coupled to the first pair of syringes 20 via manifold 22 to form the sealant delivery apparatus 10, as described above with respect to Figs. 1-7.

It is noted that a kit may be formed by a combination of the sealant delivery apparatus 10 (assembled or disassembled) of Fig. 1 and/or Fig. 2 with the pair of connecters 144-1, 144-2 and the second pair of syringes 120 of Fig. 9.

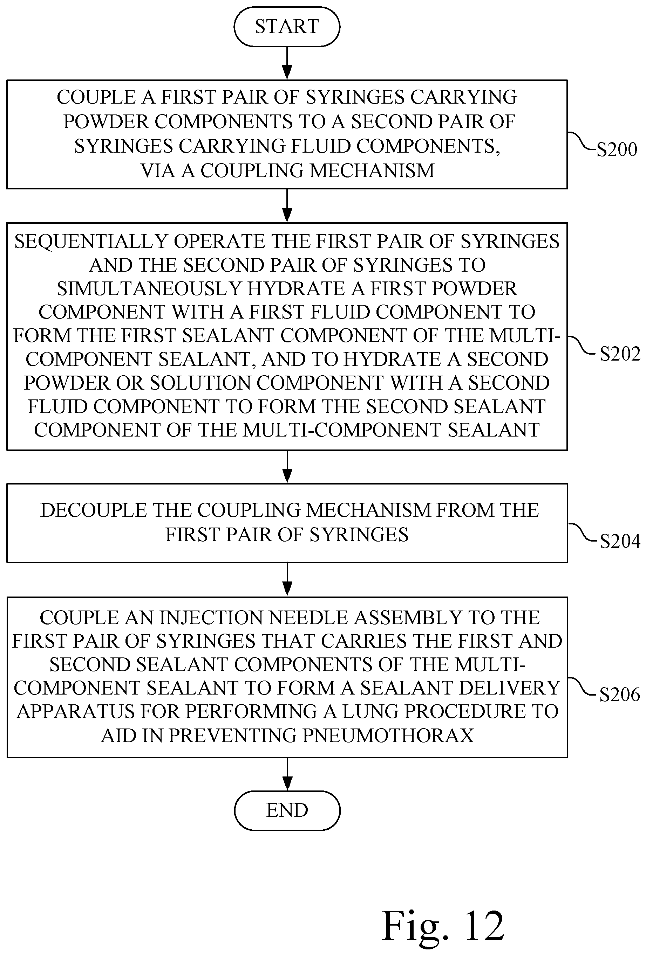

Fig. 12 is a flowchart of a method for preparing sealant delivery apparatus 10, and more particularly the pair of syringes 20 of sealant delivery apparatus 10, for use in a lung access procedure, wherein the following method steps may be sequentially performed.

At step S200, the first pair of syringes 20 are coupled to the second pair of syringes 120 via coupling mechanism 122.

At step S202, the first pair of syringes 20 and the second pair of syringes 120 are sequentially (i.e., alternatingly) operated to simultaneously hydrate powder or solution component 24-1 with fluid component 24-2 to form the first sealant component 24 of the multi-component sealant, and to hydrate powder or solution component 26-1 with fluid component 26-2 to form the second sealant component 26 of the multi-component sealant.

The step of sequentially operating the first pair of syringes 20 and the second pair of syringes 120 may be performed by alternatingly depressing second actuator 128 of the second pair of syringes 120 and first actuator 28 of the first pair of syringes 20 to alternatingly transfer any contents of first component reservoir 130 and second component reservoir 132 to first component chamber 30 and second component chamber 32, respectively, and transfer any contents of first component chamber 30 and second component chamber 32 to first component reservoir 130 and second component reservoir 132

When fully hydrated, the first sealant component 24 of the multi-component sealant resides in first component chamber 30 of the first pair of syringes 20, and the second sealant component 26 of the multi-component sealant resides in second component chamber 32 of the first pair of syringes 20. To accomplish this, a desired amount of the first sealant component 24 and the second sealant component 26 are moved into the first pair of syringes 20 by depressing the second actuator 128 of the second pair of syringes 120 until the desired amount moves into the first pair of syringes 20.

At step S204, coupling mechanism 122 may be decoupled from the first pair of syringes 20, and manifold 22 may be coupled to the first pair of syringes 20. Alternatively, coupling mechanism 122 may be decoupled from the second pair of syringes 120, and manifold 22 may be formed by coupling Y-connector 39 to coupling mechanism 122.

At step S206, injection needle assembly 14 is coupled to the first pair of syringes 20, e.g., via manifold 22 (see Fig. 1), to form the sealant delivery apparatus 10, as described above with respect to Figs. 1-7, which is now ready for performing a lung procedure to aid in preventing pneumothorax.

In a method of use of the prepared sealant delivery apparatus 10 described above at steps S200-S206, elongate hollow stylet 48 is inserted into the patient to create an access path, and actuator 28 of the pair of syringes 20 may be depressed as the access path is being created so as to deliver the multi-component sealant from the plurality of side ports 52 into the access path at the subcutaneous tissue, pleura layers, pleura space, and/or lung parenchyma as the access path is being created.

The following items also relate to the disclosure:

The disclosure, in one form, is related to a sealant delivery apparatus for a lung access procedure, in particular for prevention of pneumothorax. The sealant delivery apparatus includes a sealant applicator device and an injection needle assembly. The sealant applicator device may be configured to separately carry each of a first sealant component of a multi-component sealant and a second sealant component of the multi-component sealant. The sealant applicator device has at least one output port. The injection needle assembly has a hub and an elongate hollow stylet that extends distally from the hub. The hub may be configured for removable connection to the sealant applicator device. The elongate hollow stylet may be configured to facilitate (provide) fluid communication with the at least one output port of the sealant applicator device to receive the multi-component sealant. The elongate hollow stylet has a proximal portion and a distal portion. The distal portion has a closed distal end and a plurality of side ports proximal to (in the vicinity of) the closed distal end. The elongate hollow stylet has a side wall that surrounds a lumen, wherein the plurality of side ports radially extend from the lumen and through the side wall of the elongate hollow stylet in the distal portion. The plurality of side ports are in fluid communication with the at least one output port of the sealant applicator device.

In some embodiments, the plurality of side ports in the distal portion of the elongate hollow stylet comprises at least three side ports in the distal portion arranged around a perimeter of the elongate hollow stylet.

In some embodiments, the plurality of side ports in the distal portion of the elongate hollow stylet includes at least two longitudinally spaced side ports.

In some embodiments, the closed distal end of the elongate hollow stylet may be a closed needle tip that terminates a distal extent of the lumen.

In some embodiments, the elongate hollow stylet may comprise an elongate cannula that defines the side wall and the lumen, and a stylet needle tip may be defined by the closed distal end, wherein the stylet needle tip may be attached to the elongate cannula to distally close the lumen of the elongate cannula.

In any of the embodiments, the sealant applicator device may include a pair of syringes having an actuator, a first component chamber that may be configured to carry the first sealant component of the multi-component sealant, and a second component chamber that may be configured to carry the second sealant component of the multi-component sealant. The actuator may include a first piston and a second piston. The first piston may be positioned in the first component chamber proximal to the first sealant component, and the second piston may be positioned in the second component chamber proximal to the second sealant component. A manifold may include a first input port, a second input port, and the at least one output port, wherein the at least one output port is in fluid communication with each of the first input port and the second input port. The first input port is in fluid communication with the first component chamber and the second input port is in fluid communication with the second component chamber.

In some embodiments, the manifold may be a Y-connector that has an internal Y-passageway that includes the first input port, the second input port, and the at least one output port configured as a single output port. The single output port may be in fluid communication with the plurality of side ports in the distal end of the elongate hollow stylet having the closed distal end.

Optionally, the apparatus may comprise an introducer cannula having a cannula lumen and a distal annular rim. The cannula lumen may be configured to receive the elongate hollow stylet of the injection needle assembly, wherein when the elongate hollow stylet of the injection needle assembly is fully inserted into the cannula lumen of the introducer cannula, the plurality of side ports are located distal to the distal annular rim of the introducer cannula.

The disclosure may also relate to a lung biopsy apparatus comprising the sealant delivery apparatus as described above.

The disclosure, in another form, is related to a system for preparing a sealant delivery apparatus for a lung access procedure. The system includes a first pair of syringes associated with the sealant delivery apparatus, a second pair of syringes, and a coupling mechanism. The first pair of syringes may include a first actuator, a first component chamber having a first component port, and a second component chamber having a second component port. The first actuator may include a first piston and a second piston. The first piston is positioned in the first component chamber and the second piston is positioned in the second component chamber. The first component chamber initially contains a first powder or solution component of a first sealant component of a multi-component sealant, and the second component chamber initially contains a second powder or solution component of the second sealant component of the multi-component sealant. The second pair of syringes may include a second actuator, a first component reservoir having a first transfer port, and a second component reservoir having a second transfer port. The second actuator may include a third piston and a fourth piston. The third piston is positioned in the first component reservoir and the fourth piston is positioned in the second component reservoir. The first component reservoir initially contains a first fluid component of the first sealant component of the multi-component sealant, and the second component reservoir initially contains a second fluid component of the second sealant component of the multi-component sealant. The coupling mechanism has a first coupling end and a second coupling end. The first coupling end may be configured to releasably connect to the first pair of syringes and the second coupling end may be configured to releasably connect to the second pair of syringes. The coupling mechanism may have a first passage and a second passage. The first passage may be configured to facilitate fluid communication between the first transfer port of the second pair of syringes and the first component port of the first pair of syringes, and the second passage may be configured to facilitate fluid communication between the second transfer port of the second pair of syringes and the second component port of the first pair of syringes.

In some system embodiments, the first actuator of the first pair of syringes may be configured to simultaneously move the first piston and the second piston, and the second actuator of the second pair of syringes may be configured to simultaneously move the third piston and the fourth piston.

In any of the system embodiments, the second pair of syringes may be configured such that a first depression of the second actuator simultaneously transfers the first fluid component of the first sealant component into the first component chamber carrying the first powder or solution component of the first sealant component and transfers the second fluid component of the second sealant component into the second component chamber carrying the second powder or solution component of the second sealant component.

In any of the system embodiments, the first pair of syringes and the second pair of syringes may be configured to alternatingly transfer any contents of the first component reservoir and the second component reservoir to the first component chamber and the second component chamber, respectively, and to transfer any contents of the first component chamber and the second component chamber to the first component reservoir and the second component reservoir, respectively, by alternatingly depressing the second actuator and the first actuator.

In any of the system embodiments, the first pair of syringes and the second pair of syringes may be configured to hydrate the first powder or solution component with the first fluid component to form the first sealant component of the multi-component sealant, wherein when fully hydrated, the first sealant component of the multi-component sealant resides in the first component chamber of the first pair of syringes. Also, the first pair of syringes and the second pair of syringes may be configured to hydrate the second powder or solution component with the second fluid component to form the second sealant component of the multi-component sealant, wherein when fully hydrated, the second sealant component of the multi-component sealant resides in the second component chamber of the first pair of syringes.

Optionally, the first coupling end of the coupling mechanism may include a first snap latch and a second snap latch, and the second coupling end of the coupling mechanism may include a third snap latch and a fourth snap latch. Also, a first snap catch may be associated with the first component chamber of the first pair of syringes, wherein the first snap catch is configured to releasably engage the first snap latch of the coupling mechanism. A second snap catch may be associated with the second component chamber of the first pair of syringes, wherein the second snap catch is configured to releasably engage the second snap latch of the coupling mechanism. A third snap catch may be associated with the first component reservoir of the second pair of syringes, wherein the third snap catch is configured to releasably engage the third snap latch of the coupling mechanism. A fourth snap catch may be associated with the second component reservoir of the second pair of syringes, wherein the fourth snap catch is configured to releasably engage the fourth snap latch of the coupling mechanism.

In any of the system embodiments, the system may optionally include an injection needle assembly having a hub and an elongate hollow stylet that extends distally from the hub. The hub may be configured for removable connection to the sealant applicator device. The elongate hollow stylet may be configured to facilitate fluid communication with the first component port and the second component port of the sealant applicator device to receive the multi-component sealant. The elongate hollow stylet has a proximal portion and a distal portion. The distal portion may have a closed distal end and a plurality of side ports proximal to the closed distal end. The elongate hollow stylet may have a side wall that surrounds a lumen, wherein the plurality of side ports radially extend from the lumen and through the side wall of the elongate hollow stylet in the distal portion. The plurality of side ports are configured for fluid communication with the first component port and the second component port of the sealant applicator device.

The disclosure may also relate to a lung biopsy system comprising the system as described above.

The disclosure may also relate to a combination comprising the sealant delivery apparatus as described above and the system for preparing a sealant delivery apparatus as described above.

The disclosure, in another form, is related to a method for preparing a sealant delivery apparatus for use in a lung access procedure. The method may comprise providing a first pair of syringes that includes a first actuator, a first component chamber having a first component port, and a second component chamber having a second component port, and wherein the first actuator includes a first piston and a second piston, the first piston may be positioned in the first component chamber and the second piston may be positioned in the second component chamber, the first component chamber containing a first powder or solution component of a first sealant component of a multi-component sealant, and the second component chamber containing a second powder or solution component of the second sealant component of the multi-component sealant; providing a second pair of syringes that includes a second actuator, a first component reservoir having a first transfer port, and a second component reservoir having a second transfer port, and wherein the second actuator includes a third piston and a fourth piston, the third piston may be positioned in the first component reservoir and the fourth piston may be positioned in the second component reservoir, the first component reservoir containing a first fluid component of the first sealant component of the multi-component sealant, and the second component reservoir containing a second fluid component of the second sealant component of the multi-component sealant; and providing a coupling mechanism having a first coupling end and a second coupling end, the first coupling end may be releasably connectable to the first pair of syringes and the second coupling end may be releasably connectable to the second pair of syringes, the coupling mechanism having a first passage and a second passage, wherein the first passage facilitates fluid communication between the first transfer port of the second pair of syringes and the first component port of the first pair of syringes, and wherein the second passage facilitates fluid communication between the second transfer port of the second pair of syringes and the second component port of the first pair of syringes.

In some embodiments, the method may include the sequential steps of: connecting the first pair of syringes to the second pair of syringes via the coupling mechanism; moving the first actuator of the first pair of syringes to simultaneously move the first piston and the second piston; and moving the second actuator of the second pair of syringes to simultaneously move the third piston and the fourth piston.

In any of the method embodiments, the method may include connecting the first pair of syringes to the second pair of syringes via the coupling mechanism; and depressing the second actuator of the second pair of syringes to both transfer the first fluid component of the first sealant component from the first component reservoir into the first component chamber carrying the first powder or solution component of the first sealant component, and to simultaneously transfer the second fluid component of the second sealant component from the second component reservoir into the second component chamber carrying the second powder or solution component of the second sealant component.

In any of the method embodiments, the method may include connecting the first pair of syringes to the second pair of syringes via the coupling mechanism; sequentially operating the first pair of syringes and the second pair of syringes to hydrate the first powder or solution component with the first fluid component to form the first sealant component of the multi-component sealant, wherein when fully hydrated, the first sealant component of the multi-component sealant resides in the first component chamber of the first pair of syringes; and wherein simultaneous with the act of sequentially operating the first pair of syringes and the second pair of syringes, the first pair of syringes and the second pair of syringes operate to hydrate the second powder or solution component with the second fluid component to form the second sealant component of the multi-component sealant, wherein when fully hydrated, the second sealant component of the multi-component sealant resides in the second component chamber of the first pair of syringes.

In some of the method embodiments, the method may include alternatingly depressing the second actuator of the second pair of syringes and the first actuator of the first pair of syringes to alternatingly transfer any contents of the first component reservoir and the second component reservoir to the first component chamber and the second component chamber, respectively, and transfer any contents of the first component chamber and the second component chamber to the first component reservoir and the second component reservoir.

In some of the method embodiments, the method may include decoupling the coupling mechanism from the first pair of syringes; and coupling an injection needle assembly to the first pair of syringes.

While this disclosure has been described with respect to at least one embodiment, the present invention can be further modified within the scope of this disclosure This application is therefore intended to cover any variations, uses, or adaptations of the invention using its general principles. Further, this application is intended to cover such departures from the present disclosure as come within known or customary practice in the art to which this invention pertains and which fall within the limits of the appended claims

## Claims

1. A system (100) for preparing a sealant delivery apparatus (10) for use in a lung access procedure, comprising:
a first pair of syringes (20) that includes a first actuator (28), a first component chamber (30) having a first component port (30-1), and a second component chamber (32) having a second component port (32-1), and wherein the first actuator (28) includes a first piston (34) and a second piston (36), the first piston (34) being positioned in the first component chamber (30) and the second piston (36) being positioned in the second component chamber (32), the first component chamber (30) containing a first powder component (24-1) of a first sealant component (24) of a multi-component sealant, and the second component chamber (32) containing a second powder component (26-1) of a second sealant component (26) of the multi-component sealant;
a second pair of syringes (120) that includes a second actuator (128), a first component reservoir (130) having a first transfer port (130-1), and a second component reservoir (132) having a second transfer port (132-1), and wherein the second actuator (128) includes a third piston (134) and a fourth piston (136), the third piston (134) being positioned in the first component reservoir (130) and the fourth piston (136) being positioned in the second component reservoir (132), the first component reservoir (130) containing a first fluid component (24-2) of the first sealant component (24) of the multi-component sealant, and the second component reservoir (132) containing a second fluid component (26-2) of the second sealant component (26) of the multi-component sealant; and
a coupling mechanism (122) having a first coupling end (40-1) and a second coupling end (140-2), the first coupling end (40-1) configured to releasably connect to the first pair of syringes (20) and the second coupling end (140-2) configured to releasably connect to the second pair of syringes (120), the coupling mechanism (122) having a first passage (150-1) and a second passage (150-2), wherein the first passage (150-1) is configured to facilitate fluid communication between the first transfer port (130-1) of the second pair of syringes (120) and the first component port (30-1) of the first pair of syringes (20), and wherein the second passage (150-2) is configured to facilitate fluid communication between the second transfer port (132-1) of the second pair of syringes (120) and the second component port (32-1) of the first pair of syringes (20),
wherein the first coupling end (40-1) of the coupling mechanism (122) includes a first snap latch (146-1) and a second snap latch (146-2), and the second coupling end (40-1) of the coupling mechanism (122) includes a third snap latch (146-3) and a fourth snap latch (146-4), and further comprising:
a first connector (42-1) with a first snap catch (148-1) associated with the first component chamber (30) of the first pair of syringes (20), the first snap catch (148-1) configured to releasably engage the first snap latch (146-1) of the coupling mechanism (122);
a second connector (42-2) with a second snap catch (148-2) associated with the second component chamber (32) of the first pair of syringes (20), the second snap catch (148-2) configured to releasably engage the second snap latch (146-2) of the coupling mechanism;
a third connector (144-1) with a third snap catch (148-3) associated with the first component reservoir (130) of the second pair of syringes (120), the third snap catch (148-3) configured to releasably engage the third snap latch (146-3) of the coupling mechanism (122); and
a fourth connector (144-2) with a fourth snap catch (148-4) associated with the second component reservoir (132) of the second pair of syringes (120), the fourth snap catch (148-4) configured to releasably engage the fourth snap latch (146-4) of the coupling mechanism (122),
wherein the first connector (42-1) and the second connector (42-2) are included as separate parts, each of which is separately connectable to each of the first pair of syringes (20),
wherein the third connector (144-1) and the fourth connector (144-2) are included as separate parts, each of which is separately connectable to each of the second pair of syringes (120) and the coupling mechanism (122), and
wherein the first pair of syringes (20) and the second pair of syringes (120) are configured to alternatingly transfer any contents of the first component reservoir (130) and the second component reservoir (132) to the first component chamber (30) and the second component chamber (32), respectively, and to transfer any contents of the first component chamber (30) and the second component chamber (32) to the first component reservoir (130) and the second component reservoir (132), respectively, by alternatingly depressing the second actuator (128) and the first actuator (28).

2. The system according to claim 1, wherein the first actuator (28) of the first pair of syringes (20) is configured to simultaneously move the first piston (34) and the second piston (36), and wherein the second actuator (128) of the second pair of syringes is configured to simultaneously move the third piston (134) and the fourth piston (136).

3. The system according to any of claims 1 to 2, wherein the second pair of syringes (120) is configured such that a first depression of the second actuator (128) simultaneously transfers the first fluid component (24-2) of the first sealant component (24) into the first component chamber (30) carrying the first powder component (24-1) of the first sealant component (24) and transfers the second fluid component (26-2) of the second sealant component (26) into the second component chamber (32) carrying the second powder component (26-1) of the second sealant component (26).

4. The system according to any of claims 1 to 3, wherein the second actuator (128) and the first actuator (28) are configured to be alternatingly depressed until the respective component (24-1, 26-1) is fully hydrated by the respective fluid component (24-2, 26-2).

5. The system according to any of claims 1 to 4, wherein:
the first pair of syringes (20) and the second pair of syringes (120) are configured to hydrate the first powder component (24-1) with the first fluid component (24-2) to form the first sealant component (24) of the multi-component sealant, wherein when fully hydrated, the first sealant component (24) of the multi-component sealant resides in the first component chamber (30) of the first pair of syringes (20); and
the first pair of syringes (20) and the second pair of syringes (120) are configured to hydrate the second powder component (26-1) with the second fluid component (26-2) to form the second sealant component (26) of the multi-component sealant, wherein when fully hydrated, the second sealant component (26) of the multi-component sealant resides in the second component chamber (32) of the first pair of syringes (20).

6. The system according to any of claims 1 to 5, further comprising an injection needle assembly (14) having a hub (46) and an elongate hollow stylet (48) that extends distally from the hub (46), the hub (46) configured for removable connection to a sealant applicator device (12), the elongate hollow stylet (48) configured to facilitate fluid communication with the first component port (30-1) and the second component port (32-1) of the sealant applicator device (12) to receive the multi-component sealant, the elongate hollow stylet (48) having a proximal portion (48-1) and a distal portion (48-2), the distal portion (48-2) having a closed distal end (50) and a plurality of side ports (52) proximal to the closed distal end (50), the elongate hollow stylet (48) having a side wall (48-3) that surrounds a lumen (48-3), wherein the plurality of side ports (52) radially extend from the lumen (48-3) and through the side wall (48-3) of the elongate hollow stylet (48) in the distal portion (48-2), the plurality of side ports (52) configured for fluid communication with the first component port (30-1) and the second component port (32-1) of the sealant applicator device (12).

7. A method for preparing a sealant delivery apparatus (10) for use in a lung access procedure, comprising:
providing a first pair of syringes (20) that includes a first actuator (28), a first component chamber (30) having a first component port (30-1), and a second component chamber (32) having a second component port (32-1), and wherein the first actuator (28) includes a first piston (34) and a second piston (36), the first piston (34) being positioned in the first component chamber (30) and the second piston (36) being positioned in the second component chamber (32-1), the first component chamber (30) containing a first powder component (24-1) of a first sealant component (24) of a multi-component sealant, and the second component chamber (32) containing a second powder component (26-1) of the second sealant component (26) of the multi-component sealant;
providing a second pair of syringes (120) that includes a second actuator (128), a first component reservoir (130) having a first transfer port (130-1), and a second component reservoir (132) having a second transfer port (132-1), and wherein the second actuator (128) includes a third piston (134) and a fourth piston (136), the third piston (134) being positioned in the first component reservoir (130) and the fourth piston (136) being positioned in the second component reservoir (132), the first component reservoir (130) containing a first fluid component (24-2) of the first sealant component (24) of the multi-component sealant, and the second component reservoir (132) containing a second fluid component (26-2) of the second sealant component (26) of the multi-component sealant;
providing a coupling mechanism (122) having a first coupling end (40-1) and a second coupling end (140-2), the first coupling end (40-1) being releasably connectable to the first pair of syringes (20) and the second coupling end (140-2) being releasably connectable to the second pair of syringes (120), the coupling mechanism (122) having a first passage (150-1) and a second passage (150-2), wherein the first passage (150-1) facilitates fluid communication between the first transfer port (130-1) of the second pair of syringes (122) and the first component port (30-1) of the first pair of syringes (20), and wherein the second passage (150-2) facilitates fluid communication between the second transfer port (132-1) of the second pair of syringes (122) and the second component port (32) of the first pair of syringes (22), wherein the first coupling end (40-1) of the coupling mechanism (122) includes a first snap latch (146-1) and a second snap latch (146-2), and the second coupling end (40-1) of the coupling mechanism (122) includes a third snap latch (146-3) and a fourth snap latch (146-4);
providing a first connector (42-1) with a first snap catch (148-1) associated with the first component chamber (30) of the first pair of syringes (20), the first snap catch (148-1) configured to releasably engage the first snap latch (146-1) of the coupling mechanism (122);
providing a second connector (42-2) with a second snap catch (148-2) associated with the second component chamber (32) of the first pair of syringes (20), the second snap catch (148-2) configured to releasably engage the second snap latch (146-2) of the coupling mechanism;
providing a third connector (144-1) with a third snap catch (148-3) associated with the first component reservoir (130) of the second pair of syringes (120), the third snap catch (148-3) configured to releasably engage the third snap latch (146-3) of the coupling mechanism (122); and
providing a fourth connector (144-2) with a fourth snap catch (148-4) associated with the second component reservoir (132) of the second pair of syringes (120), the fourth snap catch (148-4) configured to releasably engage the fourth snap latch (146-4) of the coupling mechanism (122);
wherein the first connector (42-1) and the second connector (42-2) are included as separate parts, each of which is separately connectable to each of the first pair of syringes (20);
wherein the third connector (144-1) and the fourth connector (144-2) are included as separate parts, each of which is separately connectable to each of the second pair of syringes (120) and the coupling mechanism (122); and
alternatingly depressing the second actuator (128) of the second pair of syringes (120) and the first actuator (28) of the first pair of syringes (20) to alternatingly transfer any contents of the first component reservoir (130) and the second component reservoir (132) to the first component chamber (30) and the second component chamber (32), respectively, and transfer any contents of the first component chamber (30) and the second component chamber (32) to the first component reservoir (130) and the second component reservoir (132).

8. The method according to claim 7, comprising the sequential steps of:
connecting the first pair of syringes (20) to the second pair of syringes (120) via the coupling mechanism (122);
moving the first actuator (28) of the first pair of syringes (20) to simultaneously move the first piston (34) and the second piston (36); and
moving the second actuator (128) of the second pair of syringes (120) to simultaneously move the third piston (134) and the fourth piston (136).

9. The method according to claim 7, comprising:
connecting the first pair of syringes (20) to the second pair of syringes (120) via the coupling mechanism (122);
depressing the second actuator (128) of the second pair of syringes (120) to both transfer the first fluid component (24-2) of the first sealant component (24) from the first component reservoir (130) into the first component chamber (30) carrying the first powder component (24-1) of the first sealant component (24), and to simultaneously transfer the second fluid component (26-2) of the second sealant component (26) from the second component reservoir (132) into the second component chamber (32) carrying the second powder component (26-1) of the second sealant component (26).

10. The method according to claim 7, comprising:
connecting the first pair of syringes (20) to the second pair of syringes (120) via the coupling mechanism (122);
sequentially operating the first pair of syringes (20) and the second pair of syringes (120) to hydrate the first powder component (24-1) with the first fluid component (24-2) to form the first sealant component (24) of the multi-component sealant, wherein when fully hydrated, the first sealant component (24) of the multi-component sealant resides in the first component chamber (30) of the first pair of syringes (20); and
wherein simultaneous with the act of sequentially operating the first pair of syringes (20) and the second pair of syringes (120), the first pair of syringes (20) and the second pair of syringes (120) operate to hydrate the second powder component (26-1) with the second fluid component (26-2) to form the second sealant component (26) of the multi-component sealant, wherein when fully hydrated, the second sealant component (26) of the multi-component sealant resides in the second component chamber (32) of the first pair of syringes (20).

11. The method according to any of claims 8 to 10, wherein the act of alternatingly depressing the second actuator (128) of the second pair of syringes (120) and the first actuator (28) of the first pair of syringes (20) continues until the respective powder component (24-1, 26-1) is fully hydrated by the respective fluid component (24-2, 26-2).

12. The method according to any of claims 7 to 11, comprising:
decoupling the coupling mechanism (122) from the first pair of syringes (20); and
coupling the injection needle assembly (14) according to claim 6 to the first pair of syringes (20).

## Patentansprüche

1. System (100) zur Herstellung einer Vorrichtung zur Abgabe eines Versiegelungsmittels (10) zur Verwendung bei einer Lungenzugangsprozedur, umfassend:
ein erstes Paar von Spritzen (20), das einen ersten Aktuator (28), eine erste Komponentenkammer (30), aufweisend einen ersten Komponentenanschluss (30-1), und eine zweite Komponentenkammer (32), aufweisend einen zweiten Komponentenanschluss (32-1), einschließt, und wobei der erste Aktuator (28) einen ersten Kolben (34) und einen zweiten Kolben (36) einschließt, wobei der erste Kolben (34) in der ersten Komponentenkammer (30) positioniert ist und wobei der zweite Kolben (36) in der zweiten Komponentenkammer (32) positioniert ist, wobei die erste Komponentenkammer (30) eine erste Pulverkomponente (24-1) einer ersten Versiegelungsmittelkomponente (24) eines Mehrkomponentenversiegelungsmittels enthält und die zweite Komponentenkammer (32) eine zweite Pulverkomponente (26-1) einer zweiten Versiegelungsmittelkomponente (26) des Mehrkomponentenversiegelungsmittels enthält;
ein zweites Paar von Spritzen (120), das einen zweiten Aktuator (128), ein erstes Komponentenreservoir (130), aufweisend einen ersten Übertragungsanschluss (130-1), und ein zweites Komponentenreservoir (132), aufweisend einen zweiten Übertragungsanschluss (132-1), einschließt, und wobei der zweite Aktuator (128) einen dritten Kolben (134) und einen vierten Kolben (136) einschließt, wobei der dritte Kolben (134) im ersten Komponentenreservoir (130) positioniert ist und der vierte Kolben (136) im zweiten Komponentenreservoir (132) positioniert ist, wobei das erste Komponentenreservoir (130) eine erste Fluidkomponente (24-2) der ersten Versiegelungsmittelkomponente (24) des Mehrkomponentenversiegelungsmittels enthält, und wobei das zweite Komponentenreservoir (132) eine zweite Fluidkomponente (26-2) der zweiten Versiegelungsmittelkomponente (26) des Mehrkomponentenversiegelungsmittels enthält; und
einen Kopplungsmechanismus (122) aufweisend ein erstes Kopplungsende (40-1) und ein zweites Kopplungsende (140-2), wobei das erste Kopplungsende (40-1) konfiguriert ist, um lösbar mit dem ersten Paar von Spritzen (20) verbunden zu sein, und wobei das zweite Kopplungsende (140-2) konfiguriert ist, um lösbar mit dem zweiten Paar von Spritzen (120) verbunden zu sein, wobei der Kopplungsmechanismus (122) einen ersten Durchgang (150-1) und einen zweiten Durchgang (150-2) aufweist, wobei der erste Durchgang (150-1) konfiguriert ist, um die Fluidkommunikation zwischen dem ersten Übertragungsanschluss (130-1) des zweiten Paars von Spritzen (120) und dem ersten Komponentenanschluss (30-1) des ersten Paars von Spritzen (20) zu erleichtern, und wobei der zweite Durchgang (150-2) konfiguriert ist, um die Fluidkommunikation zwischen dem zweiten Übertragungsanschluss (132-1) des zweiten Paars von Spritzen (120) und dem zweiten Komponentenanschluss (32-1) des ersten Paars von Spritzen (20) zu erleichtern,
wobei das erste Kopplungsende (40-1) des Kopplungsmechanismus (122) eine erste Rastklinke (146-1) und eine zweite Rastklinke (146-2) einschließt und das zweite Kopplungsende (40-1) des Kopplungsmechanismus (122) eine dritte Rastklinke (146-3) und eine vierte Rastklinke (146-4) einschließt, und weiter umfassend:
einen ersten Verbinder (42-1) mit einem ersten Rasthaken (148-1), der mit der ersten Komponentenkammer (30) des ersten Paars von Spritzen (20) assoziiert ist, wobei der erste Rasthaken (148-1) konfiguriert ist, um lösbar in die erste Rastklinke (146-1) des Kopplungsmechanismus (122) einzugreifen;
einen zweiten Verbinder (42-2) mit einem zweiten Rasthaken (148-2), der mit der zweiten Komponentenkammer (32) des ersten Paars von Spritzen (20) assoziiert ist, wobei der zweite Rasthaken (148-2) konfiguriert ist, um lösbar in die zweite Rastklinke (146-2) des Kopplungsmechanismus einzugreifen;
einen dritten Verbinder (144-1) mit einem dritten Rasthaken (148-3), der mit dem ersten Komponentenreservoir (130) des zweiten Paars von Spritzen (120) assoziiert ist, wobei der dritte Rasthaken (148-3) konfiguriert ist, um lösbar in die dritte Rastklinke (146-3) des Kopplungsmechanismus (122) einzugreifen; und
einen vierten Verbinder (144-2) mit einem vierten Rasthaken (148-4), der mit dem zweiten Komponentenreservoir (132) des zweiten Paars von Spritzen (120) assoziiert ist, wobei der vierte Rasthaken (148-4) konfiguriert ist, um lösbar in die vierte Rastklinke (146-4) des Kopplungsmechanismus (122) einzugreifen,
wobei der erste Verbinder (42-1) und der zweite Verbinder (42-2) als separate Teile eingeschlossen sind, von denen jedes separat mit jeder des ersten Paars von Spritzen (20) verbindbar ist,
wobei der dritte Verbinder (144-1) und der vierte Verbinder (144-2) als separate Teile eingeschlossen sind, von denen jedes separat mit jedem des zweiten Paars von Spritzen (120) und dem Kopplungsmechanismus (122) verbindbar ist, und
wobei das erste Paar von Spritzen (20) und das zweite Paar von Spritzen (120) konfiguriert sind, um den Inhalt des ersten Komponentenreservoirs (130) und des zweiten Komponentenreservoirs (132) alternierend entsprechend in die erste Komponentenkammer (30) und die zweite Komponentenkammer (32) zu übertragen und den Inhalt der ersten Komponentenkammer (30) und der zweiten Komponentenkammer (32) durch alternierendes Herunterdrücken des zweiten Aktuators (128) und des ersten Aktuators (28) in entsprechend das erste Komponentenreservoir (130) und das zweite Komponentenreservoir (132) zu übertragen.

2. System nach Anspruch 1, wobei der erste Aktuator (28) des ersten Paars von Spritzen (20) konfiguriert ist, um den ersten Kolben (34) und den zweiten Kolben (36) gleichzeitig zu bewegen, und wobei der zweite Aktuator (128) des zweiten Paars von Spritzen konfiguriert ist, um den dritten Kolben (134) und den vierten Kolben (136) gleichzeitig zu bewegen.

3. System nach einem der Ansprüche 1 bis 2, wobei das zweite Paar von Spritzen (120) so konfiguriert ist, dass ein erstes Herunterdrücken des zweiten Aktuators (128) gleichzeitig die erste Fluidkomponente (24-2) der ersten Versiegelungsmittelkomponente (24) in die erste Komponentenkammer (30) überträgt, die die erste Pulverkomponente (24-1) der ersten Versiegelungsmittelkomponente (24) trägt, und die zweite Fluidkomponente (26-2) der zweiten Versiegelungsmittelkomponente (26) in die zweite Komponentenkammer (32) überträgt, die die zweite Pulverkomponente (26-1) der zweiten Versiegelungsmittelkomponente (26) trägt.

4. System nach einem der Ansprüche 1 bis 3, wobei der zweite Aktuator (128) und der erste Aktuator (28) konfiguriert sind, um abwechselnd heruntergedrückt zu werden, bis die jeweilige Komponente (24-1, 26-1) durch die jeweilige Fluidkomponente (24-2, 26-2) vollständig hydratisiert ist.

5. System nach einem der Ansprüche 1 bis 4, wobei:
das erste Paar von Spritzen (20) und das zweite Paar von Spritzen (120) konfiguriert sind, um die erste Pulverkomponente (24-1) mit der ersten Fluidkomponente (24-2) zu hydratisieren, um die erste Versiegelungsmittelkomponente (24) des Mehrkomponentenversiegelungsmittels auszubilden, wobei sich, wenn vollständig hydratisiert, die erste Versiegelungsmittelkomponente (24) des Mehrkomponentenversiegelungsmittels in der ersten Komponentenkammer (30) des ersten Paars von Spritzen (20) befindet; und
das erste Paar von Spritzen (20) und das zweite Paar von Spritzen (120) konfiguriert sind, um die zweite Pulverkomponente (26-1) mit der zweiten Fluidkomponente (26-2) zu hydratisieren, um die zweite Versiegelungsmittelkomponente (26) des Mehrkomponentenversiegelungsmittels auszubilden, wobei sich, wenn vollständig hydratisiert, die zweite Versiegelungsmittelkomponente (26) des Mehrkomponentenversiegelungsmittels in der zweiten Komponentenkammer (32) des ersten Paars von Spritzen (20) befindet.

6. System nach einem der Ansprüche 1 bis 5, weiter umfassend eine Injektionsnadelbaugruppe (14), aufweisend eine Hülse (46) und ein längliches hohles Stilett (48), das sich distal von der Hülse (46) erstreckt, wobei die Hülse (46) für eine lösbare Verbindung mit einer Dichtungsmittelapplikatoreinrichtung (12) konfiguriert ist, wobei das längliche hohle Stilett (48) konfiguriert ist, um die Fluidkommunikation mit dem ersten Komponentenanschluss (30-1) und dem zweiten Komponentenanschluss (32-1) der Dichtungsmittelapplikatoreinrichtung (12) zu erleichtern, um das Mehrkomponentendichtungsmittel zu empfangen, wobei das längliche hohle Stilett (48) einen proximalen Abschnitt (48-1) und einen distalen Abschnitt (48-2) aufweist, wobei der distale Abschnitt (48-2) ein geschlossenes distales Ende (50) und eine Vielzahl von Seitenanschlüssen (52) proximal zum geschlossenen distalen Ende (50) aufweist, wobei das längliche hohle Stilett (48) eine Seitenwand (48-3) aufweist, die ein Lumen (48-3) umgibt, wobei sich die Vielzahl von Seitenanschlüssen (52) radial vom Lumen (48-3) und durch die Seitenwand (48-3) des länglichen hohlen Stiletts (48) im distalen Abschnitt (48-2) erstreckt, wobei die Vielzahl von Seitenanschlüssen (52) für Fluidkommunikation mit dem ersten Komponentenanschluss (30-1) und dem zweiten Komponentenanschluss (32-1) der Dichtungsmittelapplikatoreinrichtung (12) konfiguriert ist.

7. Verfahren zur Herstellung einer Vorrichtung zur Abgabe eines Versiegelungsmittels (10) zur Verwendung bei einer Lungenzugangsprozedur, umfassend:
Bereitstellen eines ersten Paars von Spritzen (20), das einen ersten Aktuator (28), eine erste Komponentenkammer (30), aufweisend einen ersten Komponentenanschluss (30-1), und eine zweite Komponentenkammer (32), aufweisend einen zweiten Komponentenanschluss (32-1), einschließt, und wobei der erste Aktuator (28) einen ersten Kolben (34) und einen zweiten Kolben (36) einschließt, wobei der erste Kolben (34) in der ersten Komponentenkammer (30) positioniert ist und der zweite Kolben (36) in der zweiten Komponentenkammer (32-1) positioniert ist, wobei die erste Komponentenkammer (30) eine erste Pulverkomponente (24-1) einer ersten Versiegelungsmittelkomponente (24) eines Mehrkomponentenversiegelungsmittels enthält und die zweite Komponentenkammer (32) eine zweite Pulverkomponente (26-1) der zweiten Versiegelungsmittelkomponente (26) des Mehrkomponentenversiegelungsmittels enthält;
Bereitstellen eines zweiten Paars von Spritzen (120), das einen zweiten Aktuator (128), ein erstes Komponentenreservoir (130), aufweisend einen ersten Übertragungsanschluss (130-1), und ein zweites Komponentenreservoir (132), aufweisend einen zweiten Übertragungsanschluss (132-1), einschließt, wobei der zweite Aktuator (128) einen dritten Kolben (134) und einen vierten Kolben (136) einschließt, wobei der dritte Kolben (134) im ersten Komponentenreservoir (130) positioniert ist und der vierte Kolben (136) im zweiten Komponentenreservoir (132) positioniert ist, wobei das erste Komponentenreservoir (130) eine erste Fluidkomponente (24-2) der ersten Versiegelungsmittelkomponente (24) des Mehrkomponentenversiegelungsmittels enthält, und das zweite Komponentenreservoir (132) eine zweite Fluidkomponente (26-2) der zweiten Versiegelungsmittelkomponente (26) des Mehrkomponentenversiegelungsmittels enthält;
Bereitstellen eines Kopplungsmechanismus (122) aufweisend ein erstes Kopplungsende (40-1) und ein zweites Kopplungsende (140-2), wobei das erste Kopplungsende (40-1) lösbar mit dem ersten Paar von Spritzen (20) verbindbar ist und das zweite Kopplungsende (140-2) lösbar mit dem zweiten Paar von Spritzen (120) verbindbar ist, wobei der Kopplungsmechanismus (122) einen ersten Durchgang (150-1) und einen zweiten Durchgang (150-2) aufweist, wobei der erste Durchgang (150-1) Fluidkommunikation zwischen dem ersten Übertragungsanschluss (130-1) des zweiten Paars von Spritzen (122) und dem ersten Komponentenanschluss (30-1) des ersten Paars von Spritzen (20) erleichtert, und wobei der zweite Durchgang (150-2) Fluidkommunikation zwischen dem zweiten Übertragungsanschluss (132-1) des zweiten Paars von Spritzen (122) und dem zweiten Komponentenanschluss (32) des ersten Paars von Spritzen (22) erleichtert, wobei das erste Kopplungsende (40-1) des Kopplungsmechanismus (122) eine erste Rastklinke (146-1) und eine zweite Rastklinke (146-2) einschließt, und das zweite Kopplungsende (40-1) des Kopplungsmechanismus (122) eine dritte Rastklinke (146-3) und eine vierte Rastklinke (146-4) einschließt;
Bereitstellen eines ersten Verbinders (42-1) mit einem ersten Rasthaken (148-1), der mit der ersten Komponentenkammer (30) des ersten Paars von Spritzen (20) assoziiert ist, wobei der erste Rasthaken (148-1) konfiguriert ist, um lösbar in die erste Rastklinke (146-1) des Kopplungsmechanismus (122) einzugreifen;
Bereitstellen eines zweiten Verbinders (42-2) mit einem zweiten Rasthaken (148-2), der mit der zweiten Komponentenkammer (32) des ersten Paars von Spritzen (20) assoziiert ist, wobei der zweite Rasthaken (148-2) konfiguriert ist, um lösbar in die zweite Rastklinke (146-2) des Kopplungsmechanismus einzugreifen;
Bereitstellen eines dritten Verbinders (144-1) mit einem dritten Rasthaken (148-3), der mit dem ersten Komponentenreservoir (130) des zweiten Paars von Spritzen (120) assoziiert ist, wobei der dritte Rasthaken (148-3) konfiguriert ist, um lösbar in die dritte Rastklinke (146-3) des Kopplungsmechanismus (122) einzugreifen; und
Bereitstellen eines vierten Verbinders (144-2) mit einem vierten Rasthaken (148-4), der mit dem zweiten Komponentenreservoir (132) des zweiten Paars von Spritzen (120) assoziiert ist, wobei der vierte Rasthaken (148-4) konfiguriert ist, um lösbar in die vierte Rastklinke (146-4) des Kopplungsmechanismus (122) einzugreifen;
wobei der erste Verbinder (42-1) und der zweite Verbinder (42-2) als separate Teile eingeschlossen sind, von denen jedes separat mit jeder des ersten Paars von Spritzen (20) verbindbar ist;
wobei der dritte Verbinder (144-1) und der vierte Verbinder (144-2) als separate Teile eingeschlossen sind, von denen jedes separat mit jedem des zweiten Paars von Spritzen (120) und dem Kopplungsmechanismus (122) verbindbar ist; und
alternierendes Herunterdrücken des zweiten Aktuators (128) des zweiten Paars von Spritzen (120) und des ersten Aktuators (28) des ersten Paars von Spritzen (20), um alternierend den Inhalt des ersten Komponentenreservoirs (130) und des zweiten Komponentenreservoirs (132) in entsprechend die erste Komponentenkammer (30) und die zweite Komponentenkammer (32) zu übertragen und den Inhalt der ersten Komponentenkammer (30) und der zweiten Komponentenkammer (32) in entsprechend das erste Komponentenreservoir (130) und das zweite Komponentenreservoir (132) zu übertragen.

8. Verfahren nach Anspruch 7, umfassend die sequenziellen Schritte des:
Verbindens des ersten Paars von Spritzen (20) mit dem zweiten Paar von Spritzen (120) über den Kopplungsmechanismus (122);
Bewegens des ersten Aktuators (28) des ersten Paars von Spritzen (20), um den ersten Kolben (34) und den zweiten Kolben (36) gleichzeitig zu bewegen; und
Bewegens des zweiten Aktuators (128) des zweiten Paars von Spritzen (120), um den dritten Kolben (134) und den vierten Kolben (136) gleichzeitig zu bewegen.

9. Verfahren nach Anspruch 7, umfassend:
Verbinden des ersten Paars von Spritzen (20) mit dem zweiten Paar von Spritzen (120) über den Kopplungsmechanismus (122);
Herunterdrücken des zweiten Aktuators (128) des zweiten Paars von Spritzen (120), um sowohl die erste Fluidkomponente (24-2) der ersten Versiegelungsmittelkomponente (24) aus dem ersten Komponentenreservoir (130) in die erste Komponentenkammer (30) zu übertragen, die die erste Pulverkomponente (24-1) der ersten Versiegelungsmittelkomponente (24) trägt, als auch gleichzeitig die zweite Fluidkomponente (26-2) der zweiten Versiegelungsmittelkomponente (26) aus dem zweiten Komponentenreservoir (132) in die zweite Komponentenkammer (32) zu übertragen, die die zweite Pulverkomponente (26-1) der zweiten Versiegelungsmittelkomponente (26) trägt.

10. Verfahren nach Anspruch 7, umfassend:
Verbinden des ersten Paars von Spritzen (20) mit dem zweiten Paar von Spritzen (120) über den Kopplungsmechanismus (122);
sequenzielles Betreiben des ersten Paars von Spritzen (20) und des zweiten Paars von Spritzen (120), um die erste Pulverkomponente (24-1) mit der ersten Fluidkomponente (24-2) zu hydratisieren, um die erste Versiegelungsmittelkomponente (24) des Mehrkomponentenversiegelungsmittels auszubilden, wobei sich, wenn vollständig hydratisiert, die erste Versiegelungsmittelkomponente (24) des Mehrkomponentenversiegelungsmittels in der ersten Komponentenkammer (30) des ersten Paars von Spritzen (20) befindet; und
wobei gleichzeitig mit dem Akt des sequenziellen Betreibens des ersten Paars von Spritzen (20) und des zweiten Paars von Spritzen (120) das erste Paar von Spritzen (20) und das zweite Paar von Spritzen (120) arbeiten, um die zweite Pulverkomponente (26-1) mit der zweiten Fluidkomponente (26-2) zu hydratisieren, um die zweite Versiegelungsmittelkomponente (26) des Mehrkomponentenversiegelungsmittels auszubilden, wobei sich, wenn vollständig hydratisiert, die zweite Versiegelungsmittelkomponente (26) des Mehrkomponentenversiegelungsmittels in der zweiten Komponentenkammer (32) des ersten Paars von Spritzen (20) befindet.

11. Verfahren nach einem der Ansprüche 8 bis 10, wobei der Akt des alternierenden Herunterdrückens des zweiten Aktuators (128) des zweiten Paars von Spritzen (120) und des ersten Aktuators (28) des ersten Paars von Spritzen (20) so lange fortsetzt, bis die jeweilige Pulverkomponente (24-1, 26-1) durch die jeweilige Fluidkomponente (24-2, 26-2) vollständig hydratisiert ist.

12. Verfahren nach einem der Ansprüche 7 bis 11, umfassend:
Entkoppeln des Kopplungsmechanismus (122) vom ersten Paar von Spritzen (20);
und
Koppeln der Injektionsnadelbaugruppe (14) nach Anspruch 6 an das erste Paar von Spritzen (20).

## Revendications

1. Système (100) pour préparer un appareil (10) de distribution de produit d'étanchéité pour une utilisation dans une procédure d'accès au poumon, comprenant :
une première paire de seringues (20) qui inclut un premier actionneur (28), une première chambre (30) de composant présentant un premier orifice (30-1) de composant, et une deuxième chambre (32) de composant présentant un deuxième orifice (32-1) de composant, et dans lequel le premier actionneur (28) inclut un premier piston (34) et un deuxième piston (36), le premier piston (34) étant positionné dans la première chambre (30) de composant et le deuxième piston (36) étant positionné dans la deuxième chambre (32) de composant, la première chambre (30) de composant contenant un premier composant sous forme de poudre (24-1) d'un premier composant (24) de produit d'étanchéité d'un produit d'étanchéité à composants multiples, et la deuxième chambre (32) de composant contenant un deuxième composant sous forme de poudre (26-1) d'un deuxième composant (26) de produit d'étanchéité du produit d'étanchéité à composants multiples ;
une deuxième paire de seringues (120) qui inclut un deuxième actionneur (128), un premier réservoir (130) de composant présentant un premier orifice de transfert (130-1), et un deuxième réservoir (132) de composant présentant un deuxième orifice de transfert (132-1), et dans lequel le deuxième actionneur (128) inclut un troisième piston (134) et un quatrième piston (136), le troisième piston (134) étant positionné dans le premier réservoir (130) de composant et le quatrième piston (136) étant positionné dans le deuxième réservoir (132) de composant, le premier réservoir (130) de composant contenant un premier composant fluide (24-2) du premier composant (24) de produit d'étanchéité du produit d'étanchéité à composants multiples, et le deuxième réservoir (132) de composant contenant un deuxième composant fluide (26-2) du deuxième composant (26) de produit d'étanchéité du produit d'étanchéité à composants multiples ; et
un mécanisme de couplage (122) présentant une première extrémité de couplage (40-1) et une deuxième extrémité de couplage (140-2), la première extrémité de couplage (40-1) étant configurée pour être reliée de manière amovible à la première paire de seringues (20) et la deuxième extrémité de couplage (140-2) étant configurée pour être reliée de manière amovible à la deuxième paire de seringues (120), le mécanisme de couplage (122) présentant un premier passage (150-1) et un deuxième passage (150-2), dans lequel le premier passage (150-1) est configuré pour faciliter une communication fluidique entre le premier orifice de transfert (130-1) de la deuxième paire de seringues (120) et le premier orifice (30-1) de composant de la première paire de seringues (20), et dans lequel le deuxième passage (150-2) est configuré pour faciliter une communication fluidique entre le deuxième orifice de transfert (132-1) de la deuxième paire de seringues (120) et le deuxième orifice (32-1) de composant de la première paire de seringues (20), dans lequel la première extrémité de couplage (40-1) du mécanisme de couplage (122) inclut un premier loquet à encliquetage (146-1) et un deuxième loquet à encliquetage (146-2), et la deuxième extrémité de couplage (40-1) du mécanisme de couplage (122) inclut un troisième loquet à encliquetage (146-3) et un quatrième loquet à encliquetage (146-4), et comprenant en outre :
un premier connecteur (42-1) avec une première gâche à encliquetage (148-1) associée à la première chambre (30) de composant de la première paire de seringues (20), la première gâche à encliquetage (148-1) étant configurée pour être en prise de manière amovible avec le premier loquet à encliquetage (146-1) du mécanisme de couplage (122) ;
un deuxième connecteur (42-2) avec une deuxième gâche à encliquetage (148-2) associée à la deuxième chambre (32) de composant de la première paire de seringues (20), la deuxième gâche à encliquetage (148-2) étant configurée pour être en prise de manière amovible avec le deuxième loquet à encliquetage (146-2) du mécanisme de couplage ;
un troisième connecteur (144-1) avec une troisième gâche à encliquetage (148-3) associée au premier réservoir (130) de composant de la deuxième paire de seringues (120), la troisième gâche à encliquetage (148-3) étant configurée pour être en prise de manière amovible avec le troisième loquet à encliquetage (146-3) du mécanisme de couplage (122) ; et
un quatrième connecteur (144-2) avec une quatrième gâche à encliquetage (148-4) associée au deuxième réservoir (132) de composant de la deuxième paire de seringues (120), la quatrième gâche à encliquetage (148-4) étant configurée pour être en prise de manière amovible avec le quatrième loquet à encliquetage (146-4) du mécanisme de couplage (122),
dans lequel le premier connecteur (42-1) et le deuxième connecteur (42-2) sont inclus en tant que parties séparées, dont chacune peut être reliée séparément à chacune de la première paire de seringues (20),
dans lequel le troisième connecteur (144-1) et le quatrième connecteur (144-2) sont inclus en tant que parties séparées, dont chacune peut être reliée séparément à chacune de la deuxième paire de seringues (120) et au mécanisme de couplage (122), et
dans lequel la première paire de seringues (20) et la deuxième paire de seringues (120) sont configurées pour transférer de façon alternée tout contenu du premier réservoir (130) de composant et du deuxième réservoir (132) de composant vers la première chambre (30) de composant et la deuxième chambre (32) de composant, respectivement, et pour transférer tout contenu de la première chambre (30) de composant et de la deuxième chambre (32) de composant vers le premier réservoir (130) de composant et le deuxième réservoir (132) de composant, respectivement, en enfonçant de façon alternée le deuxième actionneur (128) et le premier actionneur (28).

2. Système selon la revendication 1, dans lequel le premier actionneur (28) de la première paire de seringues (20) est configuré pour déplacer simultanément le premier piston (34) et le deuxième piston (36), et dans lequel le deuxième actionneur (128) de la deuxième paire de seringues est configuré pour déplacer simultanément le troisième piston (134) et le quatrième piston (136).

3. Système selon l'une quelconque des revendications 1 à 2, dans lequel la deuxième paire de seringues (120) est configurée de telle sorte qu'un premier enfoncement du deuxième actionneur (128) transfère simultanément le premier composant fluide (24-2) du premier composant (24) de produit d'étanchéité dans la première chambre (30) de composant portant le premier composant sous forme de poudre (24-1) du premier composant (24) de produit d'étanchéité et transfère le deuxième composant fluide (26-2) du deuxième composant (26) de produit d'étanchéité dans la deuxième chambre (32) de composant portant le deuxième composant sous forme de poudre (26-1) du deuxième composant (26) de produit d'étanchéité.

4. Système selon l'une quelconque des revendications 1 à 3, dans lequel le deuxième actionneur (128) et le premier actionneur (28) sont configurés pour être
enfoncés de façon alternée jusqu'à ce que le composant (24-1, 26-1) respectif soit entièrement hydraté par le composant fluide (24-2, 26-2) respectif.

5. Système selon l'une quelconque des revendications 1 à 4, dans lequel :
la première paire de seringues (20) et la deuxième paire de seringues (120) sont configurées pour hydrater le premier composant sous forme de poudre (24-1) avec le premier composant fluide (24-2) pour former le premier composant (24) de produit d'étanchéité du produit d'étanchéité à composants multiples, dans lequel, lorsqu'il est entièrement hydraté, le premier composant (24) de produit d'étanchéité du produit d'étanchéité à composants multiples réside dans la première chambre (30) de composant de la première paire de seringues (20) ; et
la première paire de seringues (20) et la deuxième paire de seringues (120) sont configurées pour hydrater le deuxième composant sous forme de poudre (26-1) avec le deuxième composant fluide (26-2) pour former le deuxième composant (26) de produit d'étanchéité du produit d'étanchéité à composants multiples, dans lequel, lorsqu'il est entièrement hydraté, le deuxième composant (26) de produit d'étanchéité du produit d'étanchéité à composants multiples réside dans la deuxième chambre (32) de composant de la première paire de seringues (20).

6. Système selon l'une quelconque des revendications 1 à 5, comprenant en outre un ensemble aiguille d'injection (14) présentant un moyeu (46) et un stylet creux allongé (48) qui s'étend distalement à partir du moyeu (46), le moyeu (46) étant configuré pour une liaison amovible à un dispositif applicateur (12) de produit d'étanchéité, le stylet creux allongé (48) étant configuré pour faciliter une communication fluidique avec le premier orifice (30-1) de composant et le deuxième orifice (32-1) de composant du dispositif applicateur (12) de produit d'étanchéité pour recevoir le produit d'étanchéité à composants multiples, le stylet creux allongé (48) présentant une partie proximale (48-1) et une partie distale (48-2), la partie distale (48-2) présentant une extrémité distale fermée (50) et une pluralité d'orifices latéraux (52) proximaux à l'extrémité distale fermée (50), le stylet creux allongé (48) présentant une paroi latérale (48-3) qui entoure une lumière (48-3), dans lequel la pluralité d'orifices latéraux (52) s'étend radialement à partir de la lumière (48-3) et à travers la paroi latérale (48-3) du stylet creux allongé (48) dans la partie distale (48-2), la pluralité d'orifices latéraux (52) étant configurée pour une communication fluidique avec le premier orifice (30-1) de composant et le deuxième orifice (32-1) de composant du dispositif applicateur (12) de produit d'étanchéité.

7. Procédé pour préparer un appareil (10) de distribution de produit d'étanchéité pour une utilisation dans une procédure d'accès au poumon, comprenant :
la fourniture d'une première paire de seringues (20) qui inclut un premier actionneur (28), une première chambre (30) de composant présentant un premier orifice (30-1) de composant, et une deuxième chambre (32) de composant présentant un deuxième orifice (32-1) de composant, et dans lequel le premier actionneur (28) inclut un premier piston (34) et un deuxième piston (36), le premier piston (34) étant positionné dans la première chambre (30) de composant et le deuxième piston (36) étant positionné dans la deuxième chambre de composant (32-1), la première chambre (30) de composant contenant un premier composant sous forme de poudre (24-1) d'un premier composant (24) de produit d'étanchéité d'un produit d'étanchéité à composants multiples, et la deuxième chambre (32) de composant contenant un deuxième composant sous forme de poudre (26-1) du deuxième composant (26) de produit d'étanchéité du produit d'étanchéité à composants multiples ;
la fourniture d'une deuxième paire de seringues (120) qui inclut un deuxième actionneur (128), un premier réservoir (130) de composant présentant un premier orifice de transfert (130-1), et un deuxième réservoir (132) de composant présentant un deuxième orifice de transfert (132-1), et dans lequel le deuxième actionneur (128) inclut un troisième piston (134) et un quatrième piston (136), le troisième piston (134) étant positionné dans le premier réservoir (130) de composant et le quatrième piston (136) étant positionné dans le deuxième réservoir (132) de composant, le premier réservoir (130) de composant contenant un premier composant fluide (24-2) du premier composant (24) de produit d'étanchéité du produit d'étanchéité à composants multiples, et le deuxième réservoir (132) de composant contenant un deuxième composant fluide (26-2) du deuxième composant (26) de produit d'étanchéité du produit d'étanchéité à composants multiples ;
la fourniture d'un mécanisme de couplage (122) présentant une première extrémité de couplage (40-1) et une deuxième extrémité de couplage (140-2), la première extrémité de couplage (40-1) pouvant être reliée de manière amovible à la première paire de seringues (20) et la deuxième extrémité de couplage (140-2) pouvant être reliée de manière amovible à la deuxième paire de seringues (120), le mécanisme de couplage (122) présentant un premier passage (150-1) et un deuxième passage (150-2), dans lequel le premier passage (150-1) facilite une communication fluidique entre le premier orifice de transfert (130-1) de la deuxième paire de seringues (122) et le premier orifice (30-1) de composant de la première paire de seringues (20), et dans lequel le deuxième passage (150-2) facilite une communication fluidique entre le deuxième orifice de transfert (132-1) de la deuxième paire de seringues (122) et le deuxième orifice de composant (32) de la première paire de seringues (22), dans lequel la première extrémité de couplage (40-1) du mécanisme de couplage (122) inclut un premier loquet à encliquetage (146-1) et un deuxième loquet à encliquetage (146-2), et la deuxième extrémité de couplage (40-1) du mécanisme de couplage (122) inclut un troisième loquet à encliquetage (146-3) et un quatrième loquet à encliquetage (146-4) ;
la fourniture d'un premier connecteur (42-1) avec une première gâche à encliquetage (148-1) associée à la première chambre (30) de composant de la première paire de seringues (20), la première gâche à encliquetage (148-1) étant configurée pour être en prise de manière amovible avec le premier loquet à encliquetage (146-1) du mécanisme de couplage (122) ;
la fourniture d'un deuxième connecteur (42-2) avec une deuxième gâche à encliquetage (148-2) associée à la deuxième chambre (32) de composant de la première paire de seringues (20), la deuxième gâche à encliquetage (148-2) étant configurée pour être en prise de manière amovible avec le deuxième loquet à encliquetage (146-2) du mécanisme de couplage ;
la fourniture d'un troisième connecteur (144-1) avec une troisième gâche à encliquetage (148-3) associée au premier réservoir (130) de composant de la deuxième paire de seringues (120), la troisième gâche à encliquetage (148-3) étant configurée pour être en prise de manière amovible avec le troisième loquet à encliquetage (146-3) du mécanisme de couplage (122) ; et
la fourniture d'un quatrième connecteur (144-2) avec une quatrième gâche à encliquetage (148-4) associée au deuxième réservoir (132) de composant de la deuxième paire de seringues (120), la quatrième gâche à encliquetage (148-4) étant configurée pour être en prise de manière amovible avec le quatrième loquet à encliquetage (146-4) du mécanisme de couplage (122) ;
dans lequel le premier connecteur (42-1) et le deuxième connecteur (42-2) sont inclus en tant que parties séparées, dont chacune peut être reliée séparément à chacune de la première paire de seringues (20) ;
dans lequel le troisième connecteur (144-1) et le quatrième connecteur (144-2) sont inclus en tant que parties séparées, dont chacune peut être reliée séparément à chacune de la deuxième paire de seringues (120) et au mécanisme de couplage (122) ; et
l'enfoncement alterné du deuxième actionneur (128) de la deuxième paire de seringues (120) et du premier actionneur (28) de la première paire de seringues (20) pour transférer de façon alternée tout contenu du premier réservoir (130) de composant et du deuxième réservoir (132) de composant vers la première chambre (30) de composant et la deuxième chambre (32) de composant, respectivement, et transférer tout contenu de la première chambre (30) de composant et de la deuxième chambre (32) de composant vers le premier réservoir (130) de composant et le deuxième réservoir (132) de composant.

8. Procédé selon la revendication 7, comprenant les étapes séquentielles de liaison de la première paire de seringues (20) à la deuxième paire de seringues (120) via le mécanisme de couplage (122) ;
déplacement du premier actionneur (28) de la première paire de seringues (20) pour déplacer simultanément le premier piston (34) et le deuxième piston (36) ; et
déplacement du deuxième actionneur (128) de la deuxième paire de seringues (120) pour déplacer simultanément le troisième piston (134) et le quatrième piston (136).

9. Procédé selon la revendication 7, comprenant :
la liaison de la première paire de seringues (20) à la deuxième paire de seringues (120) via le mécanisme de couplage (122) ;
l'enfoncement du deuxième actionneur (128) de la deuxième paire de seringues (120) pour à la fois transférer le premier composant fluide (24-2) du premier composant (24) de produit d'étanchéité du premier réservoir (130) de composant dans la première chambre (30) de composant portant le premier composant sous forme de poudre (24-1) du premier composant (24) de produit d'étanchéité, et pour transférer simultanément le deuxième composant fluide (26-2) du deuxième composant (26) de produit d'étanchéité du deuxième réservoir (132) de composant dans la deuxième chambre (32) de composant portant le deuxième composant sous forme de poudre (26-1) du deuxième composant (26) de produit d'étanchéité.

10. Procédé selon la revendication 7, comprenant :
la liaison de la première paire de seringues (20) à la deuxième paire de seringues (120) via le mécanisme de couplage (122) ;
l'actionnement séquentiel de la première paire de seringues (20) et de la deuxième paire de seringues (120) pour hydrater le premier composant sous forme de poudre (24-1) avec le premier composant fluide (24-2) pour former le premier composant (24) de produit d'étanchéité du produit d'étanchéité à composants multiples, dans lequel, lorsqu'il est entièrement hydraté, le premier composant (24) de produit d'étanchéité du produit d'étanchéité à composants multiples réside dans la première chambre (30) de composant de la première paire de seringues (20) ; et
dans lequel, simultanément à l'acte d'actionnement séquentiel de la première paire de seringues (20) et de la deuxième paire de seringues (120), la première paire de seringues (20) et la deuxième paire de seringues (120) fonctionnent pour hydrater le deuxième composant sous forme de poudre (26-1) avec le deuxième composant fluide (26-2) pour former le deuxième composant (26) de produit d'étanchéité du produit d'étanchéité à composants multiples, dans lequel, lorsqu'il est entièrement hydraté, le deuxième composant (26) de produit d'étanchéité du produit d'étanchéité à composants multiples réside dans la deuxième chambre (32) de composant de la première paire de seringues (20).

11. Procédé selon l'une quelconque des revendications 8 à 10, dans lequel l'acte d'enfoncer de façon alternée le deuxième actionneur (128) de la deuxième paire de seringues (120) et le premier actionneur (28) de la première paire de seringues (20) se poursuit jusqu'à ce que le composant sous forme de poudre (24-1, 26-1) respectif soit entièrement hydraté par le composant fluide (24-2, 26-2) respectif.

12. Procédé selon l'une quelconque des revendications 7 à 11, comprenant :
le découplage du mécanisme de couplage (122) de la première paire de seringues (20) ; et le couplage de l'ensemble aiguille d'injection (14) selon la revendication 6 à la première paire de seringues (20).
